(19) Europäisches Patentamt
European Patent Office
Office européen des brevets

(11) Publication number: 0 130 735 B1

(12) EUROPEAN PATENT SPECIFICATION

(45) Date of publication of patent specification: 02.11.88

(21) Application number: 84304158.3

(22) Date of filing: 20.06.84

(51) Int. Cl.[4]: A 61 K 31/505, C 07 D 401/12, C 07 D 239/47, C 07 D 239/56, C 07 D 405/12, C 07 D 409/12, C 07 D 403/12

# (54) Amino-pyrimidine derivatives.

(30) Priority: 30.06.83 US 509886
30.06.83 US 509887

(43) Date of publication of application:
09.01.85 Bulletin 85/02

(45) Publication of the grant of the patent:
02.11.88 Bulletin 88/44

(84) Designated Contracting States:
AT BE CH DE FR GB IT LI LU NL SE

(56) References cited:
EP-A-0 005 984
AU-B-2 287 077
DD-A- 72 790
GB-A- 932 674
US-A-4 224 327

CHEMICAL ABSTRACTS, vol. 94, no. 9, March 2, 1981, Columbus, Ohio, USA A. KUMAR et al. "A novel and convenient synthesis of 2-amino-4-(N-alkyl-N-arylamino/pyrimidines" page 723, column 1, abstract-no. 65 606n

(73) Proprietor: AMERICAN HOME PRODUCTS CORPORATION
685, Third Avenue
New York, New York 10017 (US)

(72) Inventor: Bagli, Jehan Framoz
7 Cleveland Lane RD No. 4
Princeton New Jersey 08540 (US)

(74) Representative: Wileman, David Francis et al
c/o John Wyeth and Brother Limited
Huntercombe Lane South
Taplow Maidenhead Berkshire SL6 0PH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 130 735 B1

Courier Press, Leamington Spa, England.

## Description

This invention relates to pharmaceutical compositions containing aminopyrimidine derivatives, to the use of such derivatives and processes for preparing them, to the novel aminopyrimidine derivatives themselves, to intermediates useful in the preparation of the derivatives and to their preparation. The aminopyrimidine derivatives are useful as cardiotonic agents for increasing cardiac contractility.

A number of pyrimidines and 4-oxopyrimidines are described, for example, B. Rogge et al., Chem. Abstr. *81*, 25691m (1974) for East German patent 101,894, November 20, 1973; S. Kisaki et al., Chem. Pharm. Bull., *22*, 2246 (1974); Derwent Publications Ltd., Farmdoc 62457W for German Offenlegenshift 2,410,650, published September 11, 1975; Derwent Publications Ltd., Farmdoc 05783J for Japanese Patent 7,176,981, published October 10, 1982; Derwent Publications Ltd., Farmdoc 10368U for Netherland Patent 7,210,637, published February 6, 1973; Chemical Abstracts, *75*, 49129m (1971) for Japanese Patent 7,108,698, published March 5, 1971; A. Kumar et al., Synthesis, (9), 748 (1980); Derwent Publications Ltd., Farmdoc 46076R for East German Patent 72,790, published May 5, 1970; and Derwent Publications Ltd., Farmdoc 31812R for British Patent 1,189,188 published November 9, 1966, Australian Patent Specification No 502739. The pyrimidines described in the above reports are distinguished from novel compounds of this invention by the different substituents on the pyrimidine ring and the reported biological activity, if any. EP Publication No 5984 discloses tetrahdyropyrimidines as histamine $H_2$-receptor antagonists. Novel amino-pyrimidine derivatives of this invention are also distinguished from the cardiotonic pyridinones, exemplified by G. Y. Lesher et al., United States Patent 4,072,746, February 7, 1978 and G. Y. Lesher et al., United States Patent 4,313,951, February 2, 1982, by having different rings and different substitution on the rings.

Some of the 6-amino-5-pyrimidinecarbonitrile derivatives for which a novel use is claimed in this invention are described by Derwent Publications Ltd., Farmdoc 46076R for East German Patent 72,790, published May 5, 1970 as chemical intermediates for the production of physiologically active substances. Accordingly in one aspect this invention provides a compound of formula I

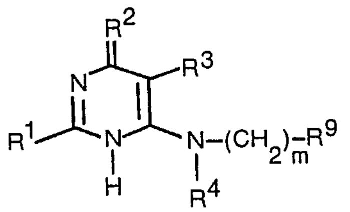

(I)

in which $R^1$ is $C_1$—$C_6$ alkyl, cyclo ($C_3$—$C_6$) alkyl or phenyl ($C_1$—$C_6$) alkyl; $R^2$ is oxo, thioxo or imino; $R^3$ is cyano, aminocarbonyl or nitro; $R^4$ is hydrogen or $C_1$—$C_4$ alkyl; $R^9$ is hydrogen, $C_2$—$C_6$ alkenyl, 1-piperidinyl, cyclo ($C_3$—$C_6$) alkyl, 2, 3 or 4-pyridinyl, 2 or 3-furanyl, 2 or 3-indolyl, 2 or 3-thienyl, 5-imidazolyl, 4-morpholinyl, phenyl optionally disubstituted with $C_1$—$C_6$ alkoxy, or 4-thiomorpholinyl; and m is an integer 0 to 2; or a therapeutically acceptable addition salt thereof for use as a pharmaceutical.

The novel compounds of this invention are represented by formula I

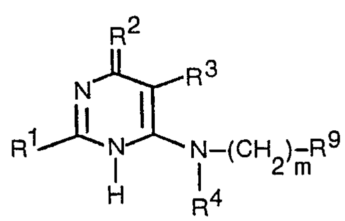

(I)

in which $R^1$ is $C_1$—$C_6$ alkyl, cyclo ($C_3$—$C_6$) alkyl or phenyl ($C_1$—$C_6$) alkyl; $R^2$ is oxo, thioxo or imino; $R^3$ is cyano, aminocarbonyl or nitro; $R^4$ is hydrogen or $C_1$—$C_4$ alkyl; $R^9$ is $C_2$—$C_6$ alkenyl, 1-piperidinyl, cyclo ($C_3$—$C_6$) alkyl, 2, 3 or 4-pyridinyl, 2 or 3-furanyl, 2 or 3-indolyl, 2 or 3-thienyl, 5-imidazolyl, 4-morpholinyl, phenyl optionally disubstituted with $C_1$—$C_6$ alkoxy or 4-thiomorpholinyl; and m is an integer 0 to 2; or a therapeutically acceptable addition salt thereof.

A preferred group of compounds of this invention is represented by formula I in which $R^1$ is $C_1$—$C_6$ alkyl, cyclo ($C_3$—$C_6$) alkyl or benzyl; $R^2$ is oxo, thioxo or imino; $R^3$ is cyano or aminocarbonyl; $R^4$ is hydrogen or $C_1$—$C_4$ alkyl; $R^9$ is $C_2$—$C_6$ alkenyl, cyclo ($C_3$—$C_6$) alkyl, 2, 3 or 4-pyridinyl, 2 or 3-furanyl, 2 or 3-indolyl, 2 or 3-thienyl, 4-morpholinyl, phenyl optionally disubstituted $C_1$—$C_6$ alkoxy; and m is an integer 0 to 2; or a therapeutically acceptable addition salt thereof.

A more preferred group of compounds of this invention is represented by formula I in which $R^1$ is $C_1$—$C_6$ alkyl, cyclo ($C_3$—$C_6$) alkyl or benzyl; $R^2$ is oxo or thioxo; $R^3$ is cyano; $R^4$ is hydrogen; $R^9$ is $C_2$—$C_6$ alkenyl, 2, 3 or 4-pyridinyl, 2-furanyl, 3-indolyl, 3-thienyl, 4-morpholinyl, phenyl optionally disubstituted with $C_1$—$C_6$ alkoxy; and m is the integer 0 to 1; or a therapeutically acceptable addition salt thereof.

This invention also relates to the use for increasing cardiac contractility of a prior reported group of compounds of formula I in which $R^1$ is $C_1$—$C_6$ alkyl, cyclo ($C_3$—$C_6$) alkyl or benzyl; $R^2$ is oxo or thioxo; $R^3$ is cyano; $R^4$ is $C_1$—$C_4$ alkyl; $R^9$ is hydrogen, and m is 0 or a therapeutically acceptable addition salt thereof.

A preferred group of compounds for increasing cardiac contractility is represented by formula I in which $R^1$ is $C_1$—$C_6$ alkyl, cyclo ($C_3$—$C_6$) alkyl or benzyl; $R^2$ is oxo; $R^3$ is cyano; $R^4$ is $C_1$—$C_4$ alkyl; $R^9$ is hydrogen and m is 0.

This invention also relates to a pharmaceutical composition comprising a compound of formula I or a therapeutically acceptable addition salt thereof and a pharmaceutically acceptable carrier.

## Detailed Description of the Invention

The term '$C_1$—$C_6$ alkyl' as used herein means straight and branched chain alkyl radicals containing from one to six carbon atoms, preferably one to four carbon atoms and includes methyl, ethyl, propyl, 1-methylethyl, butyl, 1,1-dimethyl or pentyl, unless stated otherwise.

The term '$C_2$—$C_6$ alkenyl' as used herein means straight chain alkenyl radicals containing from two to six carbon atoms and branched chain alkenyl radicals containing from three to six carbon atoms and includes ethenyl, 2-methyl-2-propenyl or 4-hexenyl.

The term 'cyclo ($C_3$—$C_6$) alkyl' as used herein means unsaturated cyclic hydrocarbon radicals containing from three to six carbon atoms and includes cyclopropyl, cyclobutyl, cyclopentyl and cyclohexyl.

The term 'lower alkanol' as used herein means both straight and branched chain alkanols containing from one to four carbon atoms and includes methanol, ethanol, 1-methylethanol or butanol.

The term 'inorganic proton acceptor' as used herein means the inorganic bases, preferably the alkali metal hydroxides, carbonates and bicarbonates, for example, sodium bicabronate, potassium carbonate, sodium hydride or sodium methoxide.

The compounds of this invention are capable of forming acid addition salts with therapeutically acceptable acids. The acid addition salts are prepared by reacting the base form of the appropriate compound of formula I with one or more equivalents, preferably with an excess, of the appropriate acid in an organic solvent, for example, diethyl ether or an ethanol-diethyl ether mixture.

These salts, when administered to a mammal, possess the same pharmacologic activities as the corresponding bases. For many purposes it is preferable to administer the salts rather than the basic compounds. Suitable acids to form these salts include the common mineral acids, e.g. hydrohalic, sulfuric or phosphoric acid; the organic acids, e.g. maleic, citric or tartaric acid; and acids which are sparingly soluble in body fluids and which impart slow-release properties to their respective salts, e.g. pamoic or tannic acid or carboxymethyl cellulose. The addition salts thus obtained are the functional equivalent of the parent base compound in respect to their therapeutic use. Hence, these addition salts are included within the scope of this invention and are limited only by the requirement that the acids employed in forming the salts be therapeutically acceptable.

The compounds of formula I in which $R^2$ is oxo or thioxo are also capable of forming addition salts with sodium or potassium. These salts are prepared by reacting the latter compounds of formula I with one or more equivalents of sodium or potassium, or a strong base of sodium or potassium, for example, sodium hydroxide, potassium t-butoxide, sodium hydride and the like. These salts, like the acid addition salts, when administered to a mammal possess the same pharmacological activities as the corresponding nonsalt compound of formula I.

The compounds of formula I or a therapeutically acceptable addition salt thereof are useful as cardiotonic agents for increasing cardiac contractility in a mammal. The cardiotonic effect is demonstrated in standard pharmacological tests, for example, in causing an increase in the contractile force of the isolated cat papillary muscle and reversal of pentobarbital-induced cardiac failure in the dog.

The compounds of formula I of this invention are used alone or in combination wth pharmacologically acceptable carriers, the proportion of which is determined by the solubility and chemical nature of the compound, chosen route of administration and standard biological practice. For example, they are administered orally in the form of suspensions or solutions or they may be injected parenterally. For parenteral administration they can be used in the form of a sterile solution containing other solutes, for example, enough saline or glucose to make the solution isotonic.

The tablet compositions contain the active ingredient in admixture with non-toxic pharmaceutical excipients known to be suitable in the manufacture of tablets. Suitable pharmaceutical excipients are, for example, starch, milk, sugar, certain types of clay and so forth. The tablets can be uncoated or they can be coated by known techniques so as to delay disintegration and absorption in the gastrointestinal tract and thereby provide a sustained action over a longer period.

The aqueous suspensions of the compounds of formula I contain the active ingredient in admixture with one or more non-toxic pharmaceutical excipients known to be suitable in the manufacture of aqueous suspensions. Suitable excipients are, for example, methylcellulose, sodium alginate, gum acacia, lecithin and so forth. The aqueous suspensions can also contain one or more preservatives, one or more coloring agents, one or more flavoring agents and one or more sweetening agents.

Non-aqueous suspensions can be formulated by suspending the active ingredient in a vegetable oil, for example, arachis oil, olive oil, sesame oil, or coconut oil, or in a mineral oil, for example liquid paraffin,

and the suspension may contain a thickening agent, for example beeswax, hard paraffin or cetyl alcohol. These compositions can also contain a sweetening agent, flavoring agent and antioxidant.

The dosage of the compounds of formula I as cardiotonic agents will vary with the form of administration and the particular compound chosen. Furthermore, it will vary with the particular host as well as the age, weight and condition of the host under treatment as well as with the nature and extent of the symptoms. Generally, treatment is initiated with small dosages substantially less than the optimum dose of the compound. Thereafter, the dosage is increased by small increments until the optimum effect under the circumstances is reached. In general, the compounds of this invention are most desirably administered at a concentration level that will generally afford effective results without causing any harmful or deleterious side effects. For example, the effective cardiotonic amount of the compounds for oral administration can usually range from about 0.05 to 50 mg per kilogram body weight per day in single or divided doses although as aforementioned variations will occur. However, a dosage level that is in the range of from about 0.2 to 20 mg per kilogram body weight per day in single or divided doses is employed most desirably for oral administration in order to achieve effective results.

The compounds of formula I also can be used to produce beneficial effects in the treatment of congestive heart failure when combined with a therapeutically effective amount of another agent commonly used in the treatment of congestive heart failure. Such agents include, for example: vasodilators, i.e. isosorbide dinitrate, captopril, nifedipine, hydralazine and prazosin; diuretics, i.e. hydrochlorothiazide, furosemide and spironolactone; and other cardiotonics, i.e. digitalis and dobutamine. A combination of the above agents can be substituted for a single agent. Suitable methods of administration, compositions and dosages of the agents are well known in the art; for instance, "Physician Desk Reference", 37 ed., Medical Economics Co., Oradell, N.J., U.S.A., 1983. When used in combination, the compound of formula I is administered as described previously.

The invention also provides processes for preparing the compounds of formula I. A first process comprises reacting a pyrimidine IV

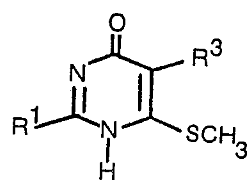

(IV)

in which $R^1$ is as defined above and $R^3$ is cyano or nitro, with an amine V

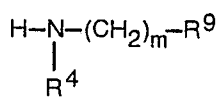

(V)

in which $R^4$, $R^9$ and m are as defined above to obtain the compound corresponding to formula I in which $R^2$ is oxo and $R^3$ is cyano or nitro and $R^1$, $R^4$, $R^9$ and m are as defined above.

A second process comprises reacting a compound of formula VI

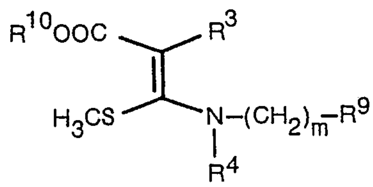

(VI)

wherein $R^3$ is cyano or nitro, $R^4$, $R^9$ and m are as defined above and $R^{10}$ is $C_1$—$C_6$ alkyl with an amidine of formula II

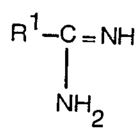

(II)

wherein $R^1$ is as defined above to give a compound of formula I wherein $R^2$ is oxo, $R^3$ is cyano or nitro, and $R^1$, $R^4$, $R^9$ and m are as defined above.

Compounds of formula I wherein $R^2$ is thioxo may be prepared by reacting a compound of formula VII

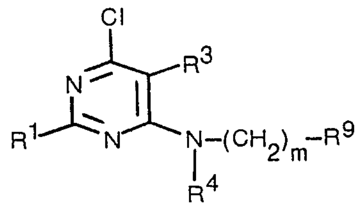

(VII)

wherein $R^1$, $R^4$, $R^9$ and m are as defined above and $R^3$ is cyano or nitro, with an aqueous ethanol solution of potassium hydroxide and hydrogen sulfide. Compounds of formula VII as defined above may be reacted with a solution of an inert organic solvent containing an excess of ammonia to give a corresponding compound of formula I wherein $R^2$ is imino.

Reaction scheme I illustrates a method for preparing the compounds of formula I

Reaction Scheme 1

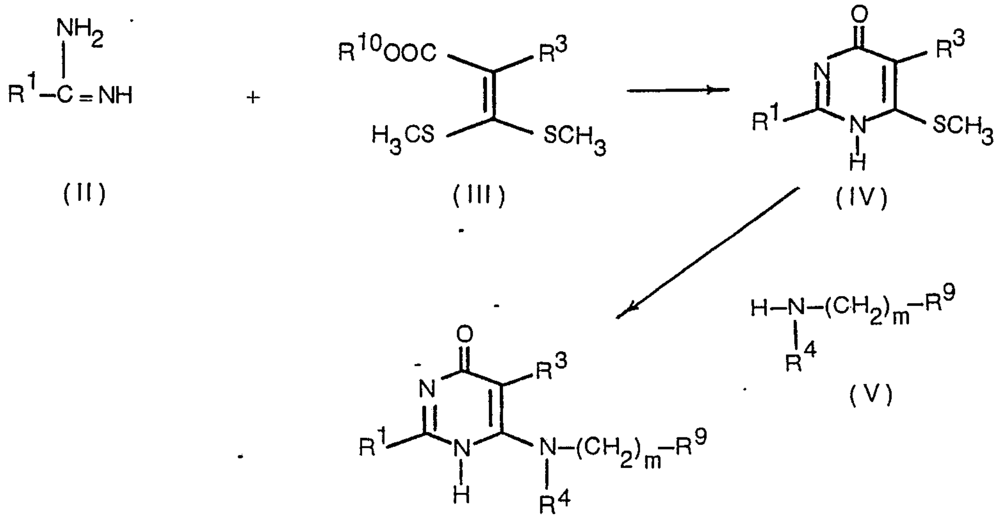

(II) + (III) (IV) (V)

I in which $R^2$ is oxo and $R^3$ is cyano or nitro.

With reference to reaction scheme 1, the amidine of formula II in which $R^1$ is as defined herein is reacted with compound of formula III in which $R^3$ is cyano or nitro, and $R^{10}$ is $C_1$—$C_6$ alkyl to obtain the pyrimidine of formula IV in which $R^1$ is as defined herein and $R^3$ is cyano or nitro, according to the method described by S. Kisaki et al, Chem. Pharm. Bull., *22*, 2246 (1974). This reaction is preferably achieved by reacting about equimolar amount of the compounds of formulae II and III in the presence of an inorganic proton acceptor, for example, sodium hydride, sodium alkoxide or potassium carbonate, in an inert organic solvent, for example, ethanol or dimethylformamide, at about 20 to 90°C for about two to ten hours.

Reaction of the pyrimidine of formula IV in which $R^1$ is as defined herein and $R^3$ is cyano or nitro, with the amine of formula V in which $R^4$, $R^9$ and m are as defined herein gives the corresponding compound of formula I in which $R^2$ is oxo, $R^3$ is cyano or nitro, and $R^1$, $R^4$, $R^9$ and m are as defined herein. About one to five equivalents of the amine of formula V are usually used and the reaction is conducted in an inert organic solvent, preferably 1,2-dimethoxyethane, at about 50 to 100°C for about 10 to 30 hours.

Reaction Scheme 2 illustrates another method for preparing some of the compounds of formula I.

Reaction Scheme 2

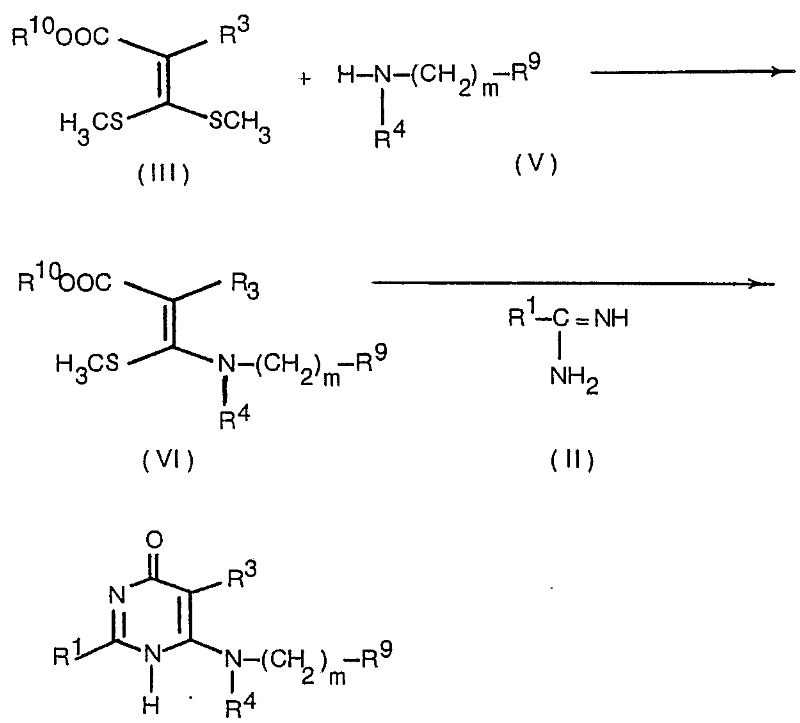

(III) (V)

(VI) (II)

I in which $R^2$ is oxo and $R^3$ is cyano or nitro.

With reference to reaction scheme 2, the compound of formula III in which $R^3$ is cyano or nitro and $R^{10}$ is $C_1$—$C_6$ alkyl is reacted with the amine of formula V in which $R^4$, $R^9$ and m are as defined herein to obtain the corresponding compound of formula VI in which $R^3$ is cyano or nitro and $R^4$, $R^9$, $R^{10}$ and m are defined herein. For this reaction, preferably about equimolar amounts of the compounds of formulae III and V are allowed to react at about 20 to 40°C for about ten minutes to two hours in an inert organic solvent, preferably dimethoxyethane, and the compound of formula VI is isolated.

Reaction of the compound of formula VI in which $R^3$ is cyano, nitro, methylsulfonyl or aminosulfonyl and $R^4$ to $R^{10}$, m and n are as defined herein with the amidine of formula II in which $R^1$ is as defined herein gives the corresponding compound of formula I in which $R^2$ is oxo; $R^3$ is cyano or nitro, and $R^1$, $R^4$, $R^9$ herein. When conducting this reaction preferably about equal molar amounts of the compounds of formula II and VI are reacted at about 75 to 150°C for about 20 to 30 hours in an inert organic solvent, preferably dimethylformamide.

To convert the compound of formula I in which $R^2$ is oxo to the corresponding compound of formula I in which $R^2$ is thioxo, the following chemical reactions are required. In the first step, the sodium salt of the compound of formula I in which $R^2$ is oxo is reacted with an excess of phosphorous oxychloride at about 90 to 120°C for about one to five hours to obtain the corresponding 4-chloropyrimidine derivative. Treatment of the latter derivative with a solution of water and ethanol containing an excess of potassium hydroxide and hydrogen sulfide at about 20 to 40°C for about 20 to 30 hours gives the corresponding compound of formula I in which $R^2$ is thioxo; $R^3$ is cyano or nitro; and $R^1$, $R^4$, $R^9$ and m are as defined herein.

Treatment of the above 4-chloropyrimidine with a solution of an inert organic solvent, preferably a lower alkanol, containing an excess of ammonia at about 50 to 80°C for about 20 to 30 hours gives the corresponding compound of formula I in which $R^2$ is imino; $R^3$ is cyano or nitro, and $R^1$, $R^4$, $R^9$ and m are as defined herein.

Hydrolysis if the compound of formula I in which $R^3$ is cyano; and $R^1$, $R^2$, $R^4$, $R^9$, m and n are as defined herein, preferably with sulfuric acid at about 60 to 100°C for one to ten hours, gives the corresponding compound of formula I in which $R^3$ is aminocarbonyl; and $R^1$, $R^2$, $R^4$, $R^9$ and m are as defined herein.

The following examples illustrate the preparation of compounds of this invention. Examples 1, 7 and 8 illustrate the preparation of intermediates. Examples 11 and 12 give test results.

Example 1

1,4-Dihydro-2-methyl-6-(methylthio)-4-oxo-5-pyrimidinecarbonitrile (IV: $R^1$ = Me and $R^3$ = cyano)

To a suspension of hexane washed sodium hydride (1.46 g, 50% in oil, 1.9 eq) in dimethylformamide (DMF, 1.5 mL) was added dropwise a solution of acetamidine hydrochloride (1.66 g, 1.1 eq) in DMF (7 mL). The reaction mixture was stirred at room temperature for 1 hr. A solution of 2-cyano-3,3-bis(methylthio)-2-propenoic acid, methyl ester (described by R. Gompper et al., Chem. Ber., *95*, 2861 (1962), 3.24 g, 1 eq) in DMF (5 mL, prepared by warming) was added dropwise. Some reaction occurred (evident by gas evolution) during addition. The reaction mixture was allowed to stir at room temperature for 4 hr and diluted with water (13 mL). After acidification with conc. hydrochloric acid (1.5 mL), the precipitate was collected and dried (2.2 g). This was combined with 2.8 g obtained from another such batch and crystallized from DMF (25 ml)/diethyl ether (30 mL), to yield the title compound (3.8 g): mp >280°C and Anal. Calcd for $C_7H_7N_3OS$: C, 46.39% H, 3.89% N, 23.19% and Found: C, 46.39% H, 3.79% N 23.27%.

Example 2 a) → q)

1,4-Dihydro-2-methyl-4-oxo-6-[(3-pyridinylmethyl)amino]-5-pyrimidinecarbnitrile (I: $R^1$ = Me, $R^2$ = O, $R^3$ = CN, $R^4$ = H, m = 1, and $R^9$ = 3-pyridinyl)

a) To a solution of 1,4-dihydro-2-methyl-6-(methylthio)-4-oxo-5-pyrimidinecarbonitrile (described in Example 1,1 eq, 4.4 g) in dimethoxyethane (33 mL) was added 3-aminomethylpyridine (9.87 mL or 10.53 g, 4 eq). The reaction mixture was refluxed for 18 hours. After about 2 hr of reflux the product started to precipitate. The reaction was cooled after 18 hr and diluted with methanol (12 mL), and the precipitate was filtered. The precipitate was washed with diethyl ether, and dried to yield white solid (5.97 g). A crystallization from hot dimethylformamide yielded the title compound: mp >260°C; Anal. Calcd for $C_{12}H_{11}H_5O$: C, 59.74% H, 4.60% N, 29.03% and found: C, 59.41% H, 4.75% N, 29.08%; IR (mineral oil) 3320, 2800, 2210, 1660 $cm^{-1}$; UV max (MeOH) 289 nm (ε 7250); 268 (8300); 263 (8200); 226 (40800); NMR (DMSO-$d_6$) δ 12.2 (1H, b), 8.5 (1H; b), 8.45 (2H, m), 7.5 (2H, m), 4.6 (2H, d), 2.25 (3H, s).

b) A suspension of the title compound (0.120 g, 1 eq) in methanol (1 mL), was stirred with methanolic hydrogen chloride (2N, 1mL, 4 eq) added to it. The reaction mixture was stirred for 1.5 hr, diluted with diethyl ether (5 mL), and filtered. The solid was washed with diethyl ether, to yield crude product (0.16 g). Crystallization from methanol-water yielded the hydrochloride salt of the title compound (0.1 g): mp >250°; and Anal. Calcd for $C_{12}H_{11}N_5O.HCl$: C, 51.89% H, 4.32% N, 25.22% and Found: C, 51.81% H, 4.38% N, 25.18%.

c) To a stirred suspension of the title compound (0.491 g, 1 eq) in methanol (2 mL), was added a solution of sodium in methanol (2N, 1.99 mL, 1 eq). The solid went into solution over a period of 0.5 hr. The mixture was stirred overnight during which time some preciptate appeared. Diethyl ether (3 mL) was added, and the sodium salt (0.58 g) was filtered. One crystallatization from methanol-diethyl ether yielded the sodium salt of the title compound (0.44 g): IR (mineral oil) 3660, 3630, 3420, 3320, 2850 $cm^{-1}$; and UV max (MeOH) 289 nm (ε 6680), 267 (7670), 263 (7570), 226 (38050).

d) → q) In the same manner, but replacing 3-aminomethylpyridine with an equivalent amount of 3-N-methylaminomethylpyridine, 2-phenylethylamine, 2-(2-pyridinyl)ethylamine, 4-aminomethylpyridine, 2-aminomethylpyridine, benzylamine, 2-aminomethylfuran, N-(2-aminoethyl)morpholine, cyclohexane-methylamine, 3-(2-aminoethyl)thiophene, 2-(3,4-dimethoxyphenyl)ethylamine, 3-(2-aminoethyl)indole, (3-pyridinyl)ethylamine, (4-pyridinyl)ethylamine, the following compounds of formula I were obtained, respectively: 1,4 - dihydro - 2 - methyl - 6 - [N - methyl - N - (3 - pyridinylmethyl)amino] - 4 - oxo - 5 - pyrimidinecarbonitrile: mp 256—258°C (cryst. from methanol); IR (mineral oil) 2900, 2200, 1645 $cm^{-1}$; UV max (MeOH) 291 nm (ε 8490), 269 (9510), 263 (8950), 234 (32200); NMR (DMSO-$d_6$) δ 11.4 (br, 1H), 8.0 (m, 4H), 4.95 (s, 2H), 3.25 (s, 3H), 2.2 (s, 3H); Anal. Calcd for $C_{13}H_{13}N_5O$: C, 61.16% H, 5.13% N, 27.44% and Found: C, 60.84% H, 5.15% , 27.37%; 1,4 - dihydro - 2 - methyl - 4 - oxo - 6 - [(phenylethyl)amino] - 5 - pyrimidinecarbonitrile: mp 264—266°C (cryst. from methanol); NMR (DMSO-$d_6$) δ 12.0 (br, 1H), 7.8 (t, 1H), 7.2 (m, 5H), 3.55 (m, 2H), 2.8 (t, 2H), 2.2 (s, 3H); Anal. Calcd for $C_{14}H_{14}N_4O$: C, 66.12% H, 5.55% N, 22.03% and Found: C, 66.11% H, 5.60% N, 21.84%; 1,4 - dihydro - 2 - methyl - 4 - oxo - 6 - [(2 - pyridinylethyl)-amino] - 5 - pyrimidinecarbonitrile: mp 276—278°C (cryst. from methanol); IR (mineral oil) 3310, 2850, 2220, 1673 $cm^{-1}$; Anal. Calcd for $C_{13}H_{13}N_5O$: C, 61.16% H, 5.13% N, 27.43% and Found: C, 60.92% H, 5.15% N, 27.15%; 1,4 - dihydro - 2 - methyl - 4 - oxo - 6 - [(4 - pyridinylmethyl)amino] - 5 - pyrimidinecarbonitrile hydrochloride: mp >250°C (cryst. from methanol-water); UV max (MeOH) 228 nm (ε 7110), 255 (8220), 225 (42790); Anal. Calcd for $C_{12}H_{11}N_5O.HCl$: C, 51.90% H, 4.35% N, 25.22% and Found: C, 51.82% H, 4.43% N, 25.36%; 1,4 - dihydro - 2 - methyl - 4 - oxo - 6 - [(2 - pyridinylmethyl)amino] - 5 - pyrimidine-carbonitrile: mp >250°C (cryst. from DMF-diethyl ether); NMR (DMSO-$d_6$) δ 2.15 (s, 3H), 4.68 (2H), 7.2 (2H), 7.7 (1H), 8.22 (1H, 8.47 (1H); Anal. Calcd for $C_{12}H_{11}N_5O$: C, 59.74% H, 4.60% N, 29.03% and Found: C, 59.69% H, 4.78%, N, 28.83%; 1,4 - dihydro - 2 - methyl - 4 - oxo - 6 - [(phenylmethyl)amino] - 5 - pyrimidinecarbonitrile: mp >290°C (cryst. from DMF); NMR (DMSO-$d_6$) δ 8.3 (t, 1H), 7.25 (s, 5H), 4.57 (d, 2H), 2.2 (s, 3H); Anal. Calcd for $C_{13}H_{12}N_4O$: C, 64.98% H, 5.04% N, 23.32% and Found: C, 64.84% H, 5.27% N, 23.28%; 1,4 - dihydro - 6 - [(2 - furanylmethyl)amino] - 2 - methyl - 4 - oxo - 5 - pyrimidinecarbonitrile: mp 297—300°C (cryst. from methanol); NMR (DMSO-$d_6$) δ 2.2 (s, 3H), 4.55 (d, 2H), 6.25 (m, 2H), 7.5 (m, 1H), 8.2 (t, 1H), 11.4 (br, 1H); Anal. Calcd for $C_{11}H_{10}N_4O_2$: C, 57.38% H, 4.38% N, 24.34% and Found: C, 57.02% H, 4.49% N, 24.15%; 1,4 - dihydro - 2 - methyl - 6 - [[2 - (4 - morpholinyl)ethyl]amino] - 4 -

oxo - 5 - pyrimidinecarbonitrile: mp 227—230°C (cryst. from methanol-acetonitrile); NMR ($CDCl_3$) δ 2.4 (s, 3H), 2.5 (m, 6H), 3.65 (m, 6H), 3.35 (t, 1H); IR (mineral oil) 3340, 2850, 1670, 1595 $cm^{-1}$; 6 - [(cyclohexylmethyl)amino] - 1,4 - dihydro - 2 - methyl - 4 - oxo - 5 - pyrimidinecarbonitrile: mp 305—308°C; NMR (DMSO-$d_6$) of sodium salt δ 1.3 (m), 1.98 (s), 3.1 (d.J = 6.5);

Anal. Calcd for $C_{13}H_{18}N_4O$: C63.39% H, 7.36% N, 22.75% and Found: C, 63.56% H, 7.32% N, 22.64%; 1,4 - dihydro - 2 - methyl - 4 - oxo - 6 - [[2 - (3 - thienyl)ethyl]amino] - 5 - pyrimidinecarbonitrile: mp 290—292°C; NMR (DMSO-$d_6$) δ 2.2 (s, 3H), 2.8 (t, 2H), 3.55 (m, 2H), 7.15 (m, 3H), 7.8 (t, 1H); Anal. Calcd for $C_{12}H_{12}N_4OS$: C, 55.36% H, 4.65% N, 21.52% and Found: C, 55.07% H, 4.61% N, 21.36%; 1,4 - dihydro - 6 - [[2 - 3,4 - dimethoxyphenyl)ethyl]amino] - 2 - methyl - 4 - oxo - 5 - pyrimidinecarbonitrile: mp 238—240°C (cryst. from methanol); NMR (DMSO-$d_6$) δ 2.2 (s, 3H), 2.7 (t, 2H), 3.5 (m, 2H), 3.7 (s, 3H), 3.73 (s, 3H), 6.75 (m, 3H), 7.7 (t, 1H); Anal. Calcd for $C_{16}H_{18}NO_3$: C61.14% H, 5.77% N, 17.82% and Found: C, 60.63% H, 5.73% N, 17.68%; 1,4 - dihydro - 6 - [[2 - (1H - indol - 3 - yl)ethyl]amino] - 2 - methyl - 4 - oxo - 5 - pyrimidinecarbonitrile: mp 297—300°C (cryst. from methanol); NMR (DMSO-$d_6$) δ 2.2 (s, 3H), 2.9 (t, 2H), 3.6 (m, 2H), 7.1 (m, 4H), 7.85 (t, 1H), 10.75 (s, 1H), 11.5 (br, 1H); Anal. Calcd for $C_{16}H_{15}N_5O$: C, 65.51% H, 5.11% N, 23.88% and Found: C, 65.14% H,5.21% N, 23.78%; 1,4 - dihydro - 2 - methyl - 4 - oxo - 6 - [2 - (3 - pyridinyl) - ethylamino] - 5 - pyrimidinecarbonitrile: mp >250°C (cryst. from DMF); UV max (MeOH) 227 nm (ε 38,300), 263 (8000), 269 (8000), 290 (6800); NMR (DMSO-$d_6$) δ 2.2 (s, 3H), 2.85 (t, 2H), 3.6 (m, 2H), 7.25 (m, 1H), 7.6 (m, 1H), 7.85 (br, 1H), 8.4 (m, 2H); Anal. Calcd for $C_{13}H_{13}N_5O$: C, 61.16% H, 5.13% N, 27.43% and Found: C, 60.62% H, 5.12% N, 27.36%; 1,4 - dihydro - 2 - methyl - 4 - oxo - 6 - [2 - (4 - pyridinyl)ethylamino] - 5 - pyrimidinecarbonitrile: mp >250°C (cryst. from DMF); UV max (MeOH) 226 nm (ε 32,700), 257 (6370), 263 (6670), 289 (6870); NMR (DMSO-$d_6$) δ 2.2 (s, 3H), 2.82 (t, 2H), 3.6 (m, 2H), 7.18 (2d, 2H), 7.83 (br, 1H), 8.42 (2d, 2H), 11.5 (br, 1H); Anal. Calcd for $C_{13}H_{13}N_5O$: C, 61.15% H, 5.13% N, 27.43% and Found: C, 61.23% H, 5.20% N, 27.41%.

## Example 3

1,4-Dihydro-2-methyl-4-oxo-6-[(2-propenyl)amino]-5-pyrimidinecarbonitrile (I: $R^1$ = Me, $R^2$ = O, $R^3$ = CN, $R^4$ = H, m = 1 and $R^9$ = ethenyl)

a) To a suspension of 2-cyano-3,3-bis(methylthio)-2-propenoic acid, methyl ester (8.12 g) in dimethoxyethane (13 mL) was added (2-propenyl)amine (2.51 g). The mixture dissolved. After about 15 min the solvent was evaporated under nitrogen and the solid residue was filtered with diethyl ether to give 2-cyano-3-methylthio-3-(2-propenyl)amino-2-propenoic acid, methyl ester (8.61 g).

Similarly, but replacing (2-propenyl)amine with 3-aminopyridine, the following compound of formula VI was obtained; 2-cyano-3-methylthio-3-(3-pyridinyl)amino-2-propenoic acid, methyl ester: mp 93—94°C.

b) To a suspension of acetamidine hydrochloride (1.25 g, 1.1 eq) in DMF (45 mL) was added 2-cyano-3-methylthio-3-(2-propenyl)amino-2-propenoic acid, methyl ester (12.54 g, 1 eq) and potassium carbonate (1.82 g, 2.2 eq). The mixture was heated to 130°C for 22 hr, cooled in an ice water bath and diluted with water (20 mL). The precipitate was collected (0.7 g). The filtrate was evaporated and suspended in a small amount of water (7 mL) and filtered to give more product (0.3 g). The product was crystallized from DMF to yield the title compound (0.44 g): mp >260°C; UV max (MeOH) 289 nm (ε 6610), 269 (5640), 227 (42640); NMR (DMSO-$d_6$) δ 2.2 (s, 3H), 3.95 (m, 2H), 5.05 (m, 2H), 5.8 (m, 1H), 7.9 (t, 1H); Anal. Calcd for $C_9H_{10}N_4O$: C, 56.84% H, 5.26% N, 29.47% and Found: C, 56.58% H, 5.34% N, 29.56%.

c) Similarly, replacement of 2 - cyano - 3 - methylthio - 3 - (2 - propenyl)amino - 2 - propenoic acid, methyl ester with 2 - cyano - 3 - methylthio - 3 - (3 - pyridinyl)amino - 2 - propenoic acid, methyl ester, the following compound of formula I was obtained: 1,4 - dihydro - 2 - methyl - 4 - oxo - 6 - [(3 - pyridinyl)amino] - 5 - pyrimidinecarbonitrile: mp >260°C (cryst. from DMF); IR (mineral oil) 3320, 2700, 2210, 1695 $cm^{-1}$; NMR (DMSO-$d_6$) δ 2.2 (s, 3H), 7.9 (m, 4H), 9.75 (br, 2H); Anal. Calcd for $C_{11}H_9N_5O$: C58.09% H, 3.99% N, 30.82% and Found: C, 57.80% H, 4.18% N, 30.36%.

## Example 4

4-Amino-2-methyl-6-[(3-pyridinylmethyl)amino]-5-pyrimidinecarbonitrile (I: $R^1$ = Me, $R^2$ = NH, $R^3$ = CN, $R^4$ = H, m = 1 and $R^9$ = 3-pyridinyl)

a) To phosphorous oxychloride (42.9 g, or 26.1 mL, 8 eq) preheated to 100°C (bath temp.) was added, the sodium salt of 1,4 - dihydro - 2 - methyl - 4 - oxo - 6 - [(3 - pyridinylmethyl)amino] - 5 - pyrimidinecarbonitrile (described in Example 2), (9.2 g, 1 eq). The reaction mixture was heated for 2 hr. The solution was cooled and poured into ice water (900 mL) with stirring. The resulting solution was basified with sodium hydroxide (50%, 100 mL). Some ethyl acetate was added, and the resulting precipitate was filtered and dried to yield a solid (4.7 g). The aqueous filtrate was extracted with ethyl acetate, and the extract was dried and evaporated to give a residue (1.8 g). The combined material was crystallized from chloroform-hexane to give 4 - chloro - 2 - methyl - 6 - [(3 - pyridinylmethyl)amino] - 5 - pyrimidinecarbonitrile: mp 165—167°C; UV max (MeOH) 305 nm (ε 4130), 254 (18100).

b) A solution of the latter compound (1.5 g, 1 eq) in methanol (25 mL) was cooled in an ice bath. Ammonia was passed into the solution for 20 min. The reaction mixture turned yellow. The mixture was heated in a closed container to 60°C for 2 hr. The precipitate began to appear concomitently with discoloration. The mixture was stirred at room temperature overnight. The solvent was removed, water was added, and the solid was filtered to give crude product (1.4 g). Three crystallizations from DMF yielded

the title compound (0.3 g): mp >280°C; UV max (MeOH) 254 nm (ε 12740), 234 (44070); Anal. Calcd for $C_{12}H_{12}N_6$: C, 59.98% H, 5.04% N, 34.98% and Found: C, 59.50% H, 5.13% N, 34.41%.

Example 5

1,4-Dihydro-2-methyl-6-[(3-pyridinylmethyl)amino]-4-thioxo-5-pyrimidinecarbonitrile (I: $R^1$ = Me, $R^2$ = S, $R^3$ = CN, $R^4$ = H, m = 1 and $R^9$ = 3-pyridinyl)

To ethanol (5 mL) was added a solution of potassium hydroxide (1.5 mL, 4 molar in water-ethanol 1:9). The mixture was cooled in ice-bath. Hydrogen sulphide was passed through the solution for 15 min. 4-Chloro - 2 - methyl - 6 - [(3 - pyridinylmethyl)amino] - 5 - pyrimidinecarbonitrile (described in Example 4, 0.78 g, 1 eq) in ethanol (7 mL) was added to the solution. The mixture was stirred at room temperature for 2.5 hr. Another portion of potassium hydroxide solution (1.5 mL) in ethanol (3.5 mL) was added. Hydrogen sulfide passed through for 15 min. It was stirred overnight at room temperature. The mixture was filtered and the precipitate was washed with some ethanol, to yield crude product (0.7 g). The solid was crystallized twice from DMF to yield the title compound (0.25 g): mp 310—312°C; NMR (DMSO-$d_6$) δ 13.2 (br, 1H), 8.8 (br, 1H), 8.5 (m, 2H), 7.5 (m, 2H), 4.6 (d, 2H); 2.3 (s, 3H); Anal. Calcd for $C_{12}H_{11}N_5S$: C, 56.01% H, 4.31% N, 27.22% and Found: C, 55.48% H, 4.49% N, 26.68%.

Example 6

1,4-Dihydro-2-methyl-4-oxo-6-[(3-pyridinylmethyl)amino]-5-pyrimidinecarboxamide (I: $R^1$ = Me, $R^2$ = O, $R^3$ = aminocarbonyl, $R^4$ = H, m = 1 and $R^9$ = 3-pyridinyl).

Sulfuric acid (4 mL) was added to 1,4 - dihydro - 2 - methyl - 4 - oxo - 6 - [(3 - pyridinylmethyl)-amino] - 5 - pyrimidinecarbonitrile (1.2 g, described in Example 2). The mixture was heated to 70°C for 4 hr. The reaction was cooled to room temperature, and carefully poured on crushed ice (75 mL). The resulting solution was neutralized with sodium hydroxide (50%, aqueous, 12.5 mL) when precipitate appeared. Filtrate after removal of precipitate showed the pH to be 4.5—5.0. Eight more drops of sodium hydroxide were added. More precipitate appeared. This was filtered off. The pH was checked and found to be 5. One more drop changed the pH to 8. The combined solid was dried and crystallized from boiling DMF to yield the title compound (1.1 g): mp 264—266°C; Anal. Calcd for $C_{12}H_{14}N_5O_2$: C, 55.38% H, 5.38% N, 26.9% and Found: C, 55.26% H, 5.12% N, 26.70%; IR (mineral oil) 3760, 3000, 1645, 1585 $cm^{-1}$; NMR (DMSO-$d_6$) δ 8.5 (1H, m), 7.55 (1H, m), 7.25 (1H, m), 9.15 (1H, m), 4.7 (2H, d), 2.22 (3H, s).

Example 7

2-Methylpropanimidamide (II: $R^1$ = 1-methylethyl)

Anhydrous hydrogen chloride was passed into a solution of 2-methylpropanenitrile (30.4 g, 40 mL) in anhydrous ethanol (25 mL) until saturation at 25°C. The stoppered reaction mixture did not crystallize after 3 days and was evaporated to dryness under reduced pressure. The oily residue crystallized after a while and was treated with a 10% solution of anhydrous ammonia in ethanol (130 mL). After stirring for 3 hr, ammonium chloride was filtered off, and the filtrate was concentrated until crystalline. The first batch of the product (24.7 g) was collected by filtration, the second batch (9.6 g) was obtained on further concentration of the filtrate to give a total yield of the hydrochloride salt of the title compound (34.3 g): mp 155—157°C.

Similarly, by replacing 2-methylpropanenitrile with propanenitrile, 2,2-dimethylpropanenitrile, cyclopropylnitrile, or 2-phenylethanenitrile, the following compounds of formula II were obtained respectively: propanimidamide hydrochloride (mp 130—132°C), 2,2-dimethylpropanimidamide hydrochloride (mp 186—188°C), cyclopropylmethanimidamide hydrochloride (mp 123—125°C), and 2-phenylethanimidamide hydrochloride (mp 145—147°C).

Example 8

1,4-Dihydro-2-(1-methylethyl)-6-(methylthio)-4-oxo-5-pyrimidinecarbonitrile (IV: $R^1$ = 1-methylethyl)

a) A solution of 2-methylpropanimidamide hydrochloride (7.0 g, described in Example 7) in dimethylformamide (15 mL) was added dropwise to a suspension of sodium hydride (3.0 g, 50% in mineral oil, prewashed with hexane) in dimethylformamide (15 mL) and the whole mixture was left at 25°C for 1 hr. 2-Cyano-3,3-bis(methylthio(-2-propenoic acid, methyl ester (6.5 g) in dimethylformamide (15 mL) was then added dropwise, and the reaction mixture was stirred for an additional 4 hr at 25°C. Water (25 mL) was then added, and acidification with concentrated hydrochloric acid (10 mL) precipitated the product. The precipitate was collected and air dried to give the title compound (6.0 g): mp >260°C.

b) → e) Similarly, by replacing 2-methylpropanimidamide with another compound of formula described in Example 7, the following compounds of formula IV were obtained respectively: 1,4 - dihydro - 2 - ethyl - 6 - (methylthio) - 4 - oxo - 5 - pyrimidinecarbonitrile (mp >260°C), 1,4 - dihydro - 2 - (1,1 - dimethylethyl) - 6 - (methylthio) - 4 - oxo - 5 - pyrimidinecarbonitrile (mp >360°C), 2-cyclopropyl - 1,4 - dihydro - 6 - (methylthio) - 4 - oxo - 5 - pyrimidinecarbonitrile [NMR (DMSO-$d_6$) δ 1.2 (d, 4H), 2.0 (m, 1H), 2.5 (s, 3H)], and 1,4 - dihydro - 6 - (methylthio) - 4 - oxo - 2 - (phenylmethyl) - 5 - pyrimidinecarbonitrile [NMR (DMSO-$d_6$) δ 2.6 (s, 3H), 4.0 (s, 2H), 7.4 (s, 5H)].

Example 9

1,4-Dihydro-2-(1-methylethyl)-4-oxo-6-[(3-pyridinylmethyl)amino]-5-pyrimidinecarbonitrile (I: $R^1$ = 1-methylethyl, $R^2$ = O, $R^3$ = CN, $R^4$ = H, m = 1 and $R^9$ = 3-pyridinyl).

a) 3-(Aminomethyl)pyridine (16 mL) was added to 1,4 - dihydro - 2 - (1 - methylethyl) - 6 - (methylthio) - 4 - oxo - 5 - pyrimidinecarbonitrile (6 g, described in Example 8) in 1,2-dimethoxyethane (50 mL), and the mixture was refluxed overnight. Evaporation to dryness under reduced pressure gave the crude product which was chromatographed over silica acid with chloroform-methanol (97:3). The product was isolated as a colorless solid which was crystallized from methanol-water to give the title compound (3.4 g): mp >260°C; NMR (DMSO-$d_6$) δ 1.1 (d, 6H), 2.7 (m, 1H), 4.55 (m, 2H), 7.3—8.4 (m, 4H), 11.5 (br, 2H); IR (mineral oil) 3335, 2900, 2210, 1660, 1585 $cm^{-1}$; Anal. Calcd for $C_{14}H_{15}N_5O$: C, 62.44% H, 5.61% N, 25.94% and Found: 62.12% H, 5.63% N, 25.92%.

b) to e) Similarly, by replacing 1,4 - dihydro - 2 - (1 - methylethyl) - 6 - (methylthio) - 4 - oxo - 5 - pyrimidinecarbonitrile with another compound of formula IV described in Example 8, the following compounds of formula I were obtained respectively: 1,4 - dihydro - 2 - ethyl - 4 - oxo - 6 - [(3 - pyridinylmethyl)amino] - 5 - pyrimidinecarbonitrile hydrochloride: mp >265°C (cryst. from methanol-water); NMR (DMSO-$d_6$) δ 1.05 (t, 3H), 2.45 (d, 2H), 4.75 (d, 2H), 6.2 (br, 2H), 8.4 (m, 4H), 11.4 (br, 1H); IR (mineral oil) 3280, 2600, 2220, 2080, 1960, 1650 $cm^{-1}$; Anal. Calcd for $C_{13}H_{14}ClN_3O$: C, 53.52% H, 4.84% N, 24.00% and Found: C, 53.17% H, 4.84% N, 23.74%; 1,4 - dihydro - 3 - (1,1 - dimethylethyl) - 4 - oxo - 6 - [(3 - pyridinylmethyl)amino] - 5 - pyrimidinecarbonitrile hydrochloride: mp >260°C (cryst. from methanol-water); NMR (DMSO-$d_6$) δ 1.17 (s, 9H), 4.15 (d, 2H), 8.5 (m, 4H), 8.6 (br, 1H), 8.9 (br, 1H), 11.9 (s, 1H); IR (mineral oil) 3280, 3180, 3100, 2600, 2220, 2100, 1970, 1660 $cm^{-1}$; Anal. Calcd for $C_{18}H_{17}N_5O$: C, 56.34% H, 5.67% N, 21.90% and Found: C, 57.74% H, 5.61% N, 21.96%; 2 - cyclopropyl - 1,4 - dihydro - 4 - oxo - 6 - [(3 - pyridinylmethyl)amino] - 5 - pyrimidinecarbonitrile: mp >260°C (cryst. from 1,2-dimethoxyethane); NMR (DMSO-$d_6$) δ 1.0 (m, 4H), 1.8 (m, 1H), 4.5 (d, 2H), 7.5—8.5 (m, 4H), 12.4 (br, 1H); IR (mineral oil 3300, 2850, 2190, 1685 $cm^{-1}$; Anal. Calcd for $C_{14}H_{13}N_5O$: C, 62.91% H, 4.90% N, 26.20% and Found: C, 62.31% H, 4.90% N, 25.98%; and 1,4 - dihydro - 4 - oxo - 2 - (phenylmethyl) - 6 - [(3 - pyrdinylmethyl)amino] - 5 - pyrimidinecarbonitrile: mp >260°C (cryst. from acetonitrile); NMR (DMSO-$d_6$) δ 3.8 (s, 2H), 4.5 (d, 2H), 7.25 (s, 5H), 7.45 and 8.4 (m, 4H), 8.4 (br, 1H); IR (mineral oil) 3330, 2800, 2210, 1660, 1590 $cm^{-1}$; Anal. Calcd for $C_{18}H_{15}N_5O$: C, 68.13% H, 4.76% N, 22.07% and Found: C, 67,83% H, 4.82% N, 21.85%.

Example 10

1,4-Dihydro-6-(ethylamino)-2-methyl-4-oxo-5-pyrimidinecarbonitrile (1: $R^1$ = Me, $R^2$ = O, $R^3$ = CN, $R^9$ = Me, m = 1.

a) To a suspension of 2-cyano-3,3-bis(methylthio-2-propenoic acid, methyl ester (8.12 g, 1 eq) in dimethoxyethane (14 mL) was added a solution of ethylamine (2.16 g, 1.2 eq.) in dimethoxyethane (6.5 mL). The suspension turned to solution, and a white solid began to precipitate. After about 10 min the solvent was removed and the residue was filtered with diethyl ether to yield 2-cyano-3-ethylamino-3-methylthio-2-propenoic acid, methyl ester (7.2 g): mp 86—88°C.

b) To a solution of acetamidine hydrochloride (0.83 g, 1.1 eq) in DMF (5.5 mL) was added potassium carbonate (1.22 g, 2.2 eq) and the mixture was stirred at room temperature for about 10 min. The above cyanoester (1.6 g, 1 eq) was added and the mixture was heated to 90°C overnight. The reaction mixture was cooled, diluted with water and filtered to give a solid (0.3 g). Further concentration to dryness followed by filtration of residue with small amount of water gave more solid (0.8 g). The two solids were pooled and crystallized from methanol to give the title compound (0.6 g): mp >290°C; Anal. Calcd for $C_8H_{10}N_4O$: C, 53.93% H, 5.61% N, 31.46% and Found: C, 53.67% H, 5.83% N, 31.31%; IR (mineral oil) 3300, 2800, 2220, 1650 $cm^{-1}$; UV λmax (MeOH) 289 nm (ε 6140), 268 (5240), 226 (40,100); NMR (DMSO-$d_6$) δ 7.75 (t, 1H), 3.4 (m, 2H), 2.23 (s, 3H), 1.1 (s, 3H).

c) to f) Similarly, the following compounds of formula I can be prepared: 1,4 - dihydro - 6 - (butylamino) - 2 - cyclohexyl - 4 - oxo - 5 - pyrimidinecarbonitrile, 1,4 - dihydro - 6 - (diethylamino) - 2 - butyl - 4 - oxo - 5 - pyrimidinecarbonitrile, 1,4 - dihydro - 6 - (N - methyl - N - propylamino) - 2 - benzyl - 4 - oxo - 5 - pyrimidinecarbonitrile, and 1,4 - dihydro - 6 - (propylamino) - 2 - cyclopropyl - 4 - oxo - 5 - pyrimidinecarbonitrile.

Example 11

Test for Cardiotonic Activity in Isolated Cat Papillary Muscle

A cat of either sex was anesthetized with Na pentobarbital, 25—30 mg/kg i.v. The heart was rapidly removed and placed in cool Tyrode's solution which had been equilibrated with 95% $O_2$—5% $CO_2$. The right ventricle was opened by cutting down the sides and around the apex so that the free wall could be folded back on the atriventricular groove. Usually at least three suitably-sized papillary muscles were found (1 mm or less in thickness). Threads were tied around the chordae tendonae and the base of the muscle just above its point of insertion into the ventricular wall. The chordae were cut, and the muscle was removed along with a small button of ventricular wall. If a sufficient number of papillary muscles werre not present, trabeculae carnae could also be used. The best ones were usually found inserting just under the valve.

The preparations were mounted in tissue baths containing Tyrode's solution at 37°C bubbled with 95% $O_2$—5% $CO_2$. One thread was affixed to a tissue holder incorporating a pair of platinum electrodes and the other thread was attached to a force displacement transducer. Initial tension placed on the preparation was 0.2 g (less for very small muscles). The preparations were stimulated with square-wave pulses, 2—4 msec. in duration and 10% above threshold voltage, at a rate of 0.5 Hz. The muscles were then gently and gradually stretched to their optimum force-length relation (at which twitch tension was maximal—further stretching did not result in any further increase in the overall magnitude of the twitch). The preparations were then allowed to equilibrate for one hour with frequent changes of the bathing fluid (10—15 min intervals). The test compound was added to the bath in 0.1 mL of vehicle and incubated with the preparation for 15 min or until peak effect was attained.

Using this method, the following representative compounds of formula I were effective for increasing the force of contraction of the papillary muscle (the amount of test compound in the bath and increase in contractility is given in the parenthesis): 1,4 - dihydro - 2 - methyl - 4 - oxo - 6 - [(3 - pyridinylmethyl)-amino] - 5 - pyrimidinecarbonitrile (at $10^{-4}$ molar increased contractility by 98%), 1,4 - dihydro - 2 - methyl - 4 - oxo - 6 - [(4 - pyridinylmethyl)amino] - 5 - pyrimidinecarbonitrile (at $10^{-4}$ molar increased contractility by 139%), 1,4 - dihydro - 2 - methyl - 4 - oxo - 6 - [(phenylethyl)amino] - 5 - pyrimidine-carbonitrile (at $10^{-4}$ molar increased contractility by 142%), 1,4 - dihydro - 2 - ethyl - 4 - oxo - 6 - [(3 - pyridinylmethyl)amino] - 5 - pyrimidinecarbonitrile (at $10^{-4}$ molar increased contractility by 140%), 1,4 - dihydro - 6 - [(2 - furanylmethyl)amino] - 2 - methyl - 4 - oxo - 5 - pyrimidinecarbonitrile (at $10^{-4}$ molar increased contractility by 108%, 2 - cyclopropyl - 1,4 - dihydro - 4 - oxo - 6 - [(3 - pyridinylmethyl)-amino] - 5 - pyrimidinecarbonitrile (at $10^{-4}$ molar increased contractility by 129%), 1,4 - dihydro - 2 - methyl - 6 - [(2 - (4 - morpholinyl)ethyl]amino] - 4 - oxo - 5 - pyrimidinecarbonitrile (at $10^{-4}$ molar increased contractility by 62%), 1,4 - dihydro - 2 - methyl - 4 - oxo - 6 - [[2 - (3 - thienyl)ethyl]amino] - 5 - pyrimidinecarbonitrile (at $10^{-4}$ molar increased contractility by 149%), 1,4 - dihydro - 2 - methyl - 4 - oxo - 6 - [(2 - propenyl)amino] - 5 - pyrimidinecarbonitrile (at $10^{-4}$ molar increased contractility by 61%) and 1,4 - dihydro - 6 - (ethylamino) - 2 - methyl - 4 - oxo - 5 - pyrimidinecarbonitrile (at $10^{-4}$ molar increased contractility by 88%).

Example 12

Pentobarbital-induced Cardiac Failure in the Dog

A dog of either sex was anesthetized with Na pentobarbital, 30—35 mg/kg i.v. The trachea was intubated and the animal was respired at a rate of 20 breaths/min (stroke volume = 15 cc/kg). Both femoral veins were cannulated. One cannula was used for infusion of pentobarbital to induce and maintain failure, the other for injection of test compounds. A cannula was inserted into the aorta via a femoral artery and the cannula was attached to a blood pressure transducer for measurement of systolic, diastolic and mean aortic blood pressure. A Millar pressure-tip catheter was inserted into the other femoral artery and advanced into the left ventricle to record intraventricular pressure and dP/dt. Subdermal needle electrodes were used to record a lead II electrocardiogram and heart rate.

Following a stabilization period of at least 30 min, experimental failure was induced by the i.v. infusion of Na pentobarbital, 0.75 mg/kg/min in 0.2 mL of saline/min, until a 40—50% decrease in peak positive dP/dt was obtained. The failure state was maintained at this level throughout the experiment by continuous infusion of Na pentobarbital, 0.11—0.15 mg/kg/min. Once the maintenance infusion was started, at least 15 min were allowed to elapse before test drugs were administered.

Test compounds were prepared in N saline. Increasingly higher doses were given i.v. at 30 min-1hr intervals in order to determine a therapeutic (50% increase in dP/dt) to toxic (appearance of arrhythmias) ratio were possible.

Using this method, the following representative compounds of formula I were effective for increasing the cardiac contractility of the heart (the amount of test compound in mg per kg of body weight administered i.v. to give a 50% increase in dP/dt is given in the parenthesis): 1,4 - dihydro - 2 - methyl - 4 - oxo - 6 - [(3 - pyridinylmethyl)amino] - 5 - pyrimidinecarbonitrile (0.06 mg/kg), 1,4 - dihydro - 2 - methyl - 4 - oxo - 6 - [(4 - pyridinylmethyl)amino] - 5 - pyrimidinecarbonitrile (0.07 mg/kg), 1,4 - dihydro - 2 - ethyl - 4 - oxo - 6 - [(3 - pyridinylmethyl)amino] - 5 - pyrimidinecarbonitrile (0.05 mg/kg), 1,4 - dihydro - 6 - [(2 - furanylmethyl)amino] - 2 - methyl - 4 - oxo - 5 - pyrimidinecarbonitrile (0.06 mg/kg), 2 - cyclopropyl - 1,4 - dihydro - 4 - oxo - 6 - [(3 - pyridinylmethyl)amino] - 5 - pyrimidinecarbonitrile (0.02 mg/kg), 1,4 - dihydro - 2 - methyl - 4 - oxo - 6 - [[2 - (3 - thienyl)ethyl]amino] - 5 - pyrimidinecarbonitrile (0.08 mg/kg), and 1,4 - dihydro - 6 - (ethylamino) - 2 - methyl - 4 - oxo - 5 - pyrimidinecarbonitrile (0.02 mg/kg).

**Claims for the Contracting States: BE CH DE FR GB IT LI LU NL SE**

1. A compound of the formula I

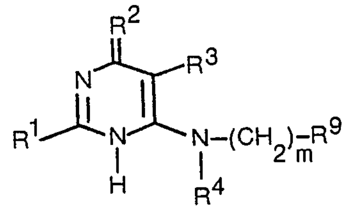

(I)

in which $R^1$ is $C_1$—$C_6$ alkyl, cyclo ($C_3$—$C_6$) alkyl or phenyl ($C_1$—$C_6$) alkyl; $R^2$ is oxo, thioxo or imino; $R^3$ is cyano, aminocarbonyl or nitro; $R^4$ is hydrogen or $C_1$—$C_4$ alkyl; $R^9$ is hydrogen, $C_2$—$C_6$ alkenyl, 1-piperidinyl, cyclo ($C_3$—$C_6$) alkyl, 2, 3 or 4-pyridinyl, 2 or 3-furanyl, 2 or 3-indolyl, 2 or 3-thienyl, 5-imidazolyl, 4-morpholinyl, phenyl optionally disubstituted with $C_1$—$C_6$ alkoxy, or 4-thiomorpholinyl; and m is an integer 0 to 2; or a therapeutically acceptable addition salt thereof for use as a pharmaceutical.

2. A pharmaceutical composition comprising a compound of formula I as defined in Claim 1 or a therapeutically acceptable addition salt thereof and a pharmaceutically acceptable carrier.

3. A cardiotonic pharmaceutical composition, which comprises a compound of the formula I

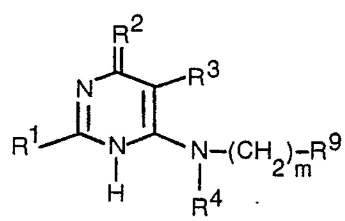

(I)

in which $R^1$ is $C_1$—$C_6$ alkyl, cyclo ($C_3$—$C_6$) alkyl or phenyl ($C_1$—$C_6$) alkyl; $R^2$ is oxo, thioxo or imino; $R^3$ is cyano, aminocarbonyl or nitro; $R^4$ is hydrogen or $C_1$—$C_4$ alkyl; $R^9$ is hydrogen, $C_2$—$C_6$ alkenyl, 1-piperidinyl, cyclo ($C_3$—$C_6$) alkyl, 2, 3 or 4-pyridinyl, 2 or 3-furanyl, 2 or 3-indolyl, 2 or 3-thienyl, 5-imidazolyl, 4-morpholinyl, phenyl optionally disubstituted with $C_1$—$C_6$ alkoxy or 4-thiomorpholinyl; and m is an integer 0 to 2; or a therapeutically acceptable addition salt thereof and an agent commonly used in treatment of congestive heart failure.

4. A cardiotonic pharmaceutical composition as claimed in claim 3 wherein said agent is selected from isosorbide dinitrate, captopril, nifedipine, hydralazine, prazosin, hydrochlorothiazide, furosemide, spironolactone, digitalis and dobutamine.

5. A compound of the formula I

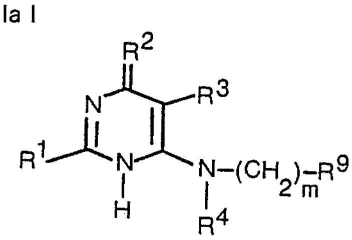

in which $R^1$ is $C_1$—$C_6$ alkyl, cyclo ($C_3$—$C_6$) alkyl or phenyl ($C_1$—$C_6$) alkyl; $R^2$ is oxo, thioxo or imino; $R^3$ is cyano, aminocarbonyl or nitro; $R^4$ is hydrogen or $C_1$—$C_4$ alkyl; $R^9$ is $C_2$—$C_6$ alkenyl, 1-piperidinyl, cyclo ($C_3$—$C_6$) alkyl, 2, 3 or 4-pyridinyl, 2 or 3-furanyl, 2 or 3-indolyl, 2 or 3-thienyl, 5-imidazolyl, 4-morpholinyl, phenyl optionally disubstituted with $C_1$—$C_6$ alkoxy, 4-thiomorpholinyl, and m is an integer 0 to 2; or a therapeutically acceptable addition salt thereof.

6. A compound as claimed in Claim 5 wherein $R^1$ is $C_1$—$C_6$ alkyl, cyclo ($C_3$—$C_6$) alkyl or benzyl; $R^2$ is oxo, thioxo or imino; $R^3$ is cyano or aminocarbonyl; $R^4$ is hydrogen or $C_1$—$C_4$ alkyl; $R^9$ is $C_2$—$C_6$ alkenyl, cyclo ($C_3$—$C_6$) alkyl, 2, 3 or 4-pyridinyl, 2 or 3-furanyl, 2 or 3-indolyl, 2 or 3-thienyl, 4-morpholinyl, phenyl optionally disubstituted with $C_1$—$C_6$ alkoxy; and m is an integer 0 to 2; or a therapeutically acceptable addition salt thereof.

7. A compound as claimed in Claim 5 wherein $R^1$ is $C_1$—$C_6$ alkyl, cyclo ($C_3$—$C_6$) alkyl or benzyl; $R^2$ is oxo or thioxo; $R^3$ is cyano; $R^4$ is hydrogen; $R^9$ is $C_2$—$C_6$ alkenyl, 2, 3 or 4-pyridinyl, 2-furanyl, 3-indolyl, 3-thienyl, 4-morpholinyl, phenyl optionally disubstituted with $C_1$—$C_6$ alkoxy; and m is 0 or 1; or a therapeutically acceptable addition salt thereof.

8. A compound as claimed in Claim 5 which is
1,4-dihydro-2-methyl-4-oxo-6-[(3-pyridinylmethyl)amino-5-pyrimidinecarbonitrile;
1,4-dihydro-2-methyl-6-[N-methyl-N-(3-pyridinylmethyl]amino]-4-oxo-5-pyrimidinecarbonitrile;
1,4-dihydro-2-methyl-4-oxo-6-[(phenylethyl)amino)-5-pyrimidinecarbonitrile;
1,4-dihydro-2-methyl-4-oxo-6-[(2-pyridinylethyl)amino]-5-pyrimidinecarbonitrile;

1,4-dihydro-2-methyl-4-oxo-6-[(4-pyridinylmethyl)amino]-5-pyrimidinecarbonitrile;
1,4-dihydro-2-methyl-4-oxo-6-[(2-pyridinylmethyl)amino]-5-pyrimidinecarbonitrile;
1,4-dihydro-2-methyl-4-oxo-6-[(phenylmethyl)amino]-5-pyrimidinecarbonitrile;
1,4-dihydro-2-methyl-4-oxo-6-[2-(3-pyridinyl)ethylamino]-5-pyrimidinecarbonitrile;
1,4-dihydro-2-methyl-4-oxo-6-[2-(4-pyridinyl)ethylamino]-5-pyrimidinecarbonitrile;
1,4-dihydro-2-methyl-4-oxo-6-[(3-pyridinyl)amino]-5-pyrimidinecarbonitrile;
1,4-dihydro-2-methyl-6-[(3-pyridinylmethyl)amino]-4-thioxo-5-pyrimidinecarbonitrile;
1,4-dihydro-2-ethyl-4-oxo-6-[(3-pyridinylmethyl)amino]-5-pyrimidinecarbonitrile;
1,4-dihydro-2-(1-methylethyl)-4-oxo-6-[(3-pyridinylmethyl)amino]-5-pyrimidine-carbonitrile;
2-cyclopropyl-1,4-dihydro-4-oxo-6-[(3-pyridinylmethyl)amino]-5-pyrimidinecarbonitrile;
1,4-dihydro-2-(1,1-dimethylethyl)-4-oxo-6-[(3-pyridinylmethyl)amino]-5-pyrimidinecarbonitrile;
1,4-dihydro-4-oxo-2-phenylmethyl-6-[(3-pyridinylmethyl)amino]-5-pyrimidinecarbonitrile;
or a pharmaceutically acceptable salt thereof.

9. A compound as claimed in Claim 5 which is
1,4-dihydro-6-[(2-furanylmethyl)amino)-2-methyl-4-oxo-5-pyrimidinecarbonitrile;
1,4-dihydro-2-methyl-6-[[2-(4-morpholinyl)ethyl]amino]-4-oxo-5-pyrimidinecarbonitrile;
1,4-dihydro-2-methyl-4-oxo-6-[[2-(3-thienyl)ethyl]amino]-5-pyrimidinecarbonitrile;
1,4-dihydro-6-[[2-(3,4-dimethoxyphenyl)ethyl]amino]-2-methyl-4-oxo-5-pyrimidine-carbonitrile;
1,4-dihydro-6-[[2-(1H-indol-3-yl)ethyl]amino]-2-methyl-4-oxo-5-pyrimidinecarbonitrile;
1,4-dihydro-2-methyl-4-oxo-6-[(2-propenyl)amino]-5-pyrimidinecarbonitrile;
or a pharmaceutically acceptable addition salt thereof.

10. A process for preparing a compound of formula I

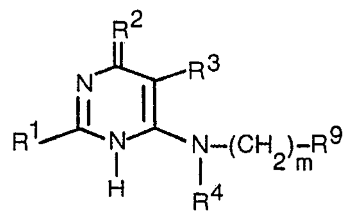

(I)

in which $R^1$ is $C_1$—$C_6$ alkyl, cyclo ($C_3$—$C_6$) alkyl or phenyl ($C_1$—$C_6$) alkyl; $R^2$ is oxo, thioxo or imino; $R^3$ is cyano, aminocarbonyl or nitro; $R^4$ each independently is hydrogen or $C_1$—$C_4$ alkyl; $R^9$ is hydrogen, $C_2$—$C_6$ alkenyl, 1-piperidinyl, cyclo ($C_3$—$C_6$) alkyl, 2, 3 or 4-pyridinyl, 2 or 3-furanyl, 2 or 3-indolyl, 2 or 3-thienyl, 5-imidazolyl, 4-morpholinyl, phenyl optionally disubstituted with $C_1$—$C_6$ alkoxy, or 4-thiomorpholinyl; and m is an integer 0 to 2; or a therapeutically acceptable addition salt thereof which comprises:

(a) reacting a pyrimidine IV

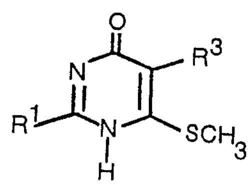

(IV)

in which $R^1$ is as defined above and $R^3$ is cyano or nitro, with an amine V

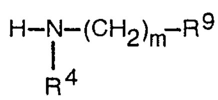

(V)

in which $R^4$, $R^9$ and m are as defined above to obtain the compound corresponding to formula I in which $R^2$ is oxo and $R^3$ is cyano or nitro, and $R^1$, $R^4$, $R^9$ and m are as defined above, or

(b) reacting a compound of formula III

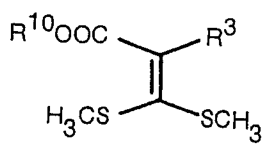

(III)

in which $R^3$ is cyano or nitro, and $R^{10}$ is $C_1$—$C_6$ alkyl with an amine V

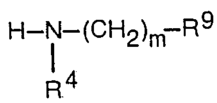

(V)

in which $R^4$, $R^9$ and m are as defined above to obtain the compound VI

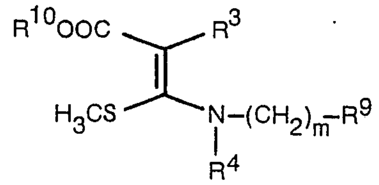

(VI)

in which $R^3$ is cyano or nitro, and $R^4$, $R^9$, $R^{10}$ and m are as defined above, and reacting the compound VI with an amidine II

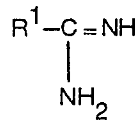

(II)

in which $R^1$ is as defined above to give a compound corresponding to formula I in which $R^2$ is oxo, $R^3$ is cyano or nitro, and $R^1$, $R^4$, $R^9$ and m are as defined above.

(c) if desired converting a compound of formula I in which $R^2$ is oxo to a compound corresponding to formula I in which $R^2$ is thioxo by reacting a sodium salt of a compound of formula I in which $R^2$ is oxo with phosphorous oxychloride to obtain a 4-chloropyrimidine of formula VII

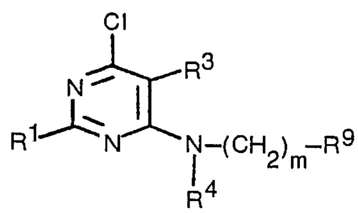

(VII)

in which $R^3$ is cyano or nitro, and $R^1$, $R^4$, $R^9$ and m are as defined above and treating the 4-chloropyrimidine VII with an aqueous ethanol solution of potassium hydroxide and hydrogen sulfide to obtain the compound corresponding to formula I in which $R^2$ is thioxo, $R^3$ is cyano or nitro, and $R^1$, $R^4$, $R^9$ and m are as defined above,

(d) if desired, treating a 4-chloropyrimidine VII with a solution of an inert organic solvent containing an excess of ammonia to obtain the compound corresponding to formula I in which $R^2$ is imino, $R^3$ is cyano or nitro, and $R^1$, $R^4$, $R^9$ and m are as defined above.

(e) if desired, hydrolyzing a compound of formula I in which $R^3$ is cyano; and $R^1$, $R^2$, $R^4$, $R^9$ and m are as defined above, with sulfuric acid to obtain the compound corresponding to formula I in which $R^3$ is aminocarbonyl; and $R^1$, $R^2$, $R^4$, $R^9$ and m are as defined above.

11. A process for preparing a compound of formula I as claimed in Claim 5 which comprises:

a) reacting a compound of formula VI

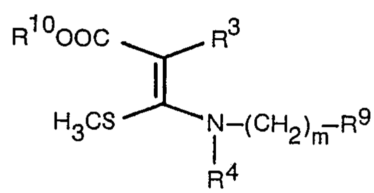

(VI)

wherein $R^3$ is cyano or nitro, and $R^4$, $R^9$ and m are as defined in Claim 5, and $R^{10}$ is $C_1$—$C_6$ alkyl with an amidine of formula II

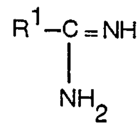

(II)

wherein $R^1$ is as defined in Claim 5 to give a compound of formula I wherein $R^2$ is oxo, $R^3$ is cyano or nitro and $R^1$, $R^4$, $R^9$ and m are as defined in Claim 5, or

b) reacting a compound of formula VII

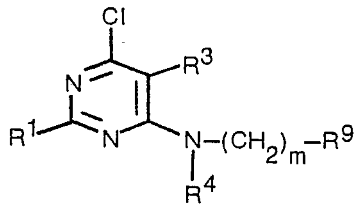

(VII)

wherein $R^1$, $R^4$, $R^9$ and m are as defined in Claim 5 and $R^3$ is cyano or nitro, with an aqueous ethanol solution of potassium hydroxide and hydrogen sulfide to give a corresponding compound of formula I wherein $R^2$ is thioxo, or

c) reacting a compound of formula VII as defined above with a solution of an inert organic solvent containing an excess of ammonia to give a corresponding compound of formula I wherein $R^2$ is imino, or

d) hydrolysing a compound of formula I as defined in Claim 5 wherein $R^3$ is cyano to give a compound of formula I wherein $R^3$ is aminocarbonyl or

e) converting a compound of formula I as defined in Claim 5 to a therapeutically acceptable salt thereof.

**Claims for the Contracting State: AT**

1. A process for preparing a compound of the formula I

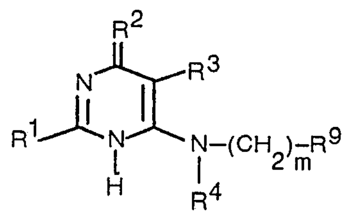

(I)

in which $R^1$ is $C_1$—$C_6$ alkyl, cyclo ($C_3$—$C_6$) alkyl-or phenyl ($C_1$—$C_6$) alkyl; $R^2$ is oxo, thioxo or imino; $R^3$ is cyano, aminocarbonyl or nitro; $R^4$ is hydrogen or $C_1$—$C_4$alkyl; $R^9$ is hydrogen, $C_2$—$C_6$ alkenyl, 1-piperidinyl, cyclo ($C_3$—$C_6$) alkyl, 2,3 or 4-pyridinyl,- 2 or 3-furanyl, 2 or 3-indolyl, 2 or 3-thienyl, 5-imidazolyl, 4-morpholinyl, phenyl, optionally disubstituted with $C_1$—$C_6$alkoxy, or 4-thiomorpholinyl; and m is an integer 0 to 2; or a therapeutically acceptable addition salt thereof, which comprises:

(a) reacting a pyrimidine IV

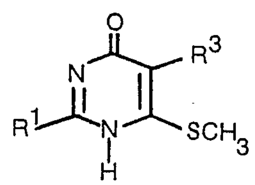

(IV)

in which $R^1$ is as defined above and $R^3$ is cyano or nitro, with an amine V

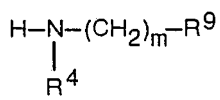

(V)

in which $R^4$, $R^9$ and m are as defined above to obtain the compound corresponding to formula I in which $R^2$ is oxo and $R^3$ is cyano or nitro, and $R^1$, $R^4$, $R^9$ and m is as defined above, or

(b) reacting a compound of formula III

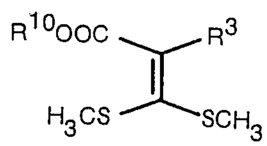

(III)

in which $R^3$ is cyano or nitro, and $R^{10}$ si $C_1$—$C_6$ alkyl with an amine V

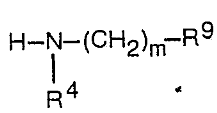

(V)

in which $R^4$, $R^9$ and m are as defined above to obtain the compound VI

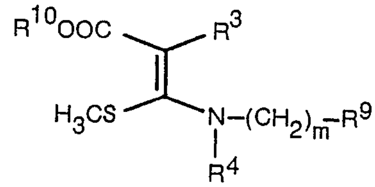

(VI)

in which $R^3$ is cyano or nitro and $R^4$, $R^9$, $R^{10}$ and m are as defined above, and reacting the compound VI with an amidine II

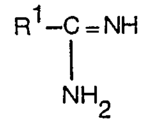

(II)

in which $R^1$ is as defined above to give a compound corresponding to formula I in which $R^2$ is oxo, $R^3$ is cyano or nitro, and $R^1$, $R^4$, $R^9$ and m are as defined above,

(c) if desired converting a compound of formula I in which $R^2$ is oxo to a compound corresponding to formula I in which $R^2$ is thioxo by reacting a sodium salt of a compound of formula I in which $R^2$ is oxo with phosphorous oxychloride to obttain a 4-choropyrimidine of formula VII

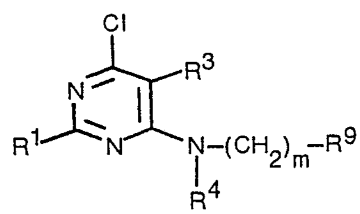

(VII)

in which $R^3$ is cyano or nitro and $R^1$, $R^4$, $R^9$ and m are as defined above and treating the 4-chloropyrimidine VII with an aqueous ethanol solution of potassium hydroxide and hydrogen sulfide to obtain the compound corresponding to formula I in which $R^2$ is thioxo, $R^3$ is cyano or nitro and $R^1$, $R^4$, $R^9$ and m are as defined above,

(d) if desired, treating a 4-chloropyrimidine VII with a solution of an inert organic solvent containing an excess of ammonia to obtain the compound corresponding to formula I in which $R^2$ is imino, $R^3$ is cyano or nito, and $R^1$, $R^4$, $R^9$ and m are as defined above,

(e) if desired, hydrolyzing a compound of formula I in which $R^3$ is cyano; and $R^1$, $R^2$, $R^4$, $R^9$ and m are as defined above, with sulfuric acid to obtain the compound corresponding to formula I in which $R^3$ is aminocarbonyl; and $R^1$, $R^2$, $R^4$, $R^9$, m and n are as defined above.

2. A process for preparing a compound of formula I

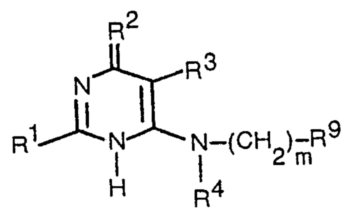

(I)

in which $R^1$ is $C_1$—$C_6$ alkyl, cyclo ($C_3$—$C_6$) alkyl or phenyl ($C_1$—$C_6$) alkyl; $R^2$ is oxo, thioxo or imino; $R^3$ is cyano, aminocarbonyl or nitro; $R^4$ is hydrogen or $C_1$—$C_6$ alkyl; $R^9$ is $C_2$—$C_6$ alkenyl, 1-piperidinyl, cyclo ($C_3$—$C_6$) alkyl, 2,3 or 4-pyridinyl, 2 or 3-furanyl, 2 or 3-indolyl, 2 or 3-thienyl, 5-imidazolyl, 4-morpholinyl, phenyl optionally disubstituted with $C_1$—$C_6$ alkoxy, 4-thiomorpholinyl and m is an integer 0 to 2; or a therapeutically acceptable addition salt thereof; which comprises

a) reacting a compound of formula VI

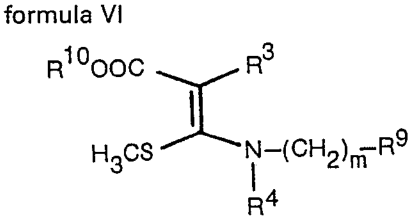

(VI)

wherein $R^3$ is cyano or nitro, and $R^4$, $R^9$ and m are as defined above and $R^{10}$ is $C_1$—$C_6$ alkyl with an amidine of formula II

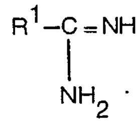

(II)

wherein $R^1$ is as defined hereinabove to give a compound of formula I wherein $R^2$ is oxo, $R^3$ is cyano or nitro and $R^1$, $R^4$, $R^9$ and m are as defined hereinabove, or

b) reacting a compound of formula VII

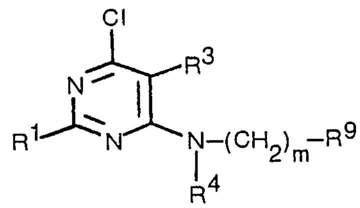

(VII)

wherein $R^1$, $R^4$, $R^9$ and m are as defined hereinabove and $R^3$ is cyano or nitro, with an aqueous ethanol solution of potassium hydroxide and hydrogen sulfide to give a corresponding compound of formula I wherein X is thioxo, or

c) reacting a compound of formula VII as defined above with a solution of an inert organic solvent containing an excess of ammonia to give a corresponding compound of formula I wherein $R^2$ is imino, or

d) hydrolysing a compound of formula I as defined hereinabove wherein $R^3$ is cyano to give a compound of formula I wherein $R^3$ is aminocarbonyl or

e) converting a compound of formula I as defined hereinabove to a therapeutically acceptable salt thereof.

3. A process as claimed in Claim 1 for preparing a compound of formula I wherein $R^1$ is $C_1$—$C_6$ alkyl, cyclo ($C_3$—$C_6$) alkyl or benzyl; $R^2$ is oxo, thioxo or imino; $R^3$ is cyano or aminocarbonyl; $R^4$ is hydrogen or $C_1$—$C_4$ alkyl; $R^9$ is $C_2$—$C_6$ alkenyl, cyclo ($C_3$—$C_6$) alkyl, 2,3 or 4-pyridinyl, 2 or 3-furanyl, 2 or 3-indolyl, 2 or 3-thienyl, 4-morpholinyl, phenyl optionally disubstituted with $C_1$—$C_6$ alkoxy; and m is an integer 0 to 2; or a therapeutically acceptable acid salt thereof.

4. A process as claimed in Claim 1 for preparing in compound of formula in which $R^1$ is $C_1$—$C_6$ alkyl, cyclo ($C_3$—$C_3$) alkyl or benzyl; $R^2$ is oxo or thioxo, $R^3$ is cyano; $R^4$ is hydrogen; $R^9$ is $C_2$—$C_6$ alkenyl, 2,3 or 4-pyridinyl, 2-furanyl, 3-indolyl, 3-thienyl, 4-morpholinyl, phenyl optionally disubstituted with $C_1$—$C_6$alkoxy; and m is 0 or 1; or a therapeutically acceptable addition salt thereof.

5. A process as claimed in Claim 1 in which the compound of formula I prepared is
1,4-dihydro-2-methyl-4-oxo-6-[(3-pyridinylmethyl)amino-5-pyrimidinecarbonitrile;
1,4-dihydro-2-methyl-6-[N-methyl-N-(3-pyridinylmethyl)amino]-4-oxo-5-pyrimidinecarbonitrile;
1,4-dihydro-2-methyl-4-oxo-6-[(phenylethyl)amino]-5-pyrimidinecarbonitrile;
1,4-dihydro-2-methyl-4-oxo-6-[(2-pyridinylethyl)amino]-5-pyrimidinecarbonitrile;
1,4-dihydro-2-methyl-4-oxo-6-[(4-pyridinylmethyl)amino]-5-pyrimidinecarbonitrile;
1,4-dihydro-2-methyl-4-oxo-6-[(2-pyridinylmethyl)amino]-5-pyrimidinecarbonitrile;
1,4-dihydro-2-methyl-4-oxo-6-[(phenylmethyl)amino]-5-pyrimidinecarbonitrile;
1,4-dihydro-2-methyl-4-oxo-6-[2-(3-pyridinyl)ethylamino]-5-pyrimidinecarbonitrile;
1,4-dihydro-2-methyl-4-oxo-6-[2-(4-pyridinyl)ethylamino]-5-pyrimidinecarbonitrile;
1,4-dihydro-2-methyl-4-oxo-6-[(3-pyrimidinyl)amino]-5-pyrimidinecarbonitrile;
1,4-dihydro-2-methyl-6-[(3-pyridinylmethyl)amino]-4-thioxo-5-pyrimidinecarbonitrile;
1,4-dihydro-2-ethyl-4-oxo-6-[(3-pyridinylmethyl)amino]-5-pyrimidinecarbonitrile;
1,4-dihydro-2-(1-methylethyl)-4-oxo-6-[(3-pyridinylmethyl)amino]-5-pyrimidine-carbonitrile;
2-cyclopropyl-1,4-dihydro-4-oxo-6-[(3-pyridinylmethyl)amino]-5-pyrimidinecarbonitrile;
1,4-dihydro-2-(1,1-dimethylethyl)-4-oxo-6-[(3-pyridinylmethyl)amino]-5-primidinecarbonitrile;
1,4-dihydro-4-oxo-2-phenylmethyl-6-[(3-pyridinylmethyl)amino]-5-pyrimidinecarbonitrile; or a pharmaceutically acceptable salt thereof.

6. A process as claimed in Claim 1 in which the compound of formula I prepared is
1,4-dihydro-6-[(2-furanylmethyl)amino]-2-methyl-4-oxo-5-pyrimidinecarbonitrile;
1,4-dihydro-2-methyl-6-[[2-(4-morpholinyl)ethyl]amino]-4-oxo-5-pyrimidinecarbonitrile;
1,4-dihydro-2-methyl-4-oxo-6-[[2-(3-thienyl)ethyl]amino]-5-pyrimidinecarbonitrile;
1,4-dihydro-6-[[2-(3,4-dimethoxyphenyl)ethyl]amino]-2-methyl-4-oxo-5-pyrimidine-carbonitrile;
1,4-dihydro-6-[[2-(1H-indol-3-yl)ethyl]amino]-2-methyl-4-oxo-5-pyrimidinecarbonitrile;
1,4-dihydro-2-methyl-4-oxo-6-[(2-propenyl)amino]-5-pyrimidinecarbonitrile; or a pharmaceutically acceptable addition salt thereof.

7. A process for preparing a pharmaceutical composition which comprises bring a compound of formula I as defined in Claim 1 or a therapeutically acceptable salt thereof into a form suitable for therapeutic administration.

8. A process as claimed in Claim 7 in which the composition also comprises an agent commonly used in treatment of congestive heart failure.

9. A process as claimed in Claim 8 in which said agent is selected from isosorbide dinitrate, captopril, nifedipine, hydralazine, prazosin, hydrochlorothiazide, furosemide, spironolactone, digitalis and dobutamine.

**Patentansprüche für die Vertragsstaaten: BE CH DE FR GB IT LI LU NL SE**

1. Verbindung der Formel (I)

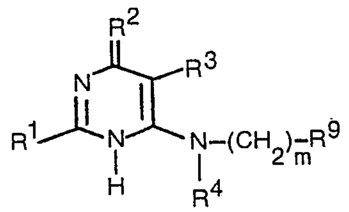

(I)

worin $R^1$ $C_1$—$C_6$-Alkyl, Cyclo($C_3$—$C_6$)alkyl oder Phenyl($C_1$—$C_6$)alkyl bedeutet; $R^2$ Oxo, Thioxo oder Imino darstellt; $R^3$ Cyano, Aminocarbonyl oder Nitro ist; $R^4$ Wasserstoff oder $C_1$—$C_4$-Alkyl bedeutet; $R^9$ Wasserstoff, $C_2$—$C_6$-Alkenyl, 1-Piperidinyl, Cyclo($C_3$—$C_6$)alkyl, 2-, 3- oder 4-Pyridinyl, 2- oder 3-Furanyl, 2- oder 3-Indolyl, 2- oder 3-Thienyl, 5-Imidazolyl, 4-Morpholinyl, Phenyl gegebenenfalls disubstituiert mit $C_1$—$C_6$-Alkoxy oder 4-Thiomorpholinyl darstellt und m eine ganze Zahl von Null bis 2 ist, oder ein therapeutisch annehmbares Additionssalz hievon zur Verwendung als Pharmazeutikum.

2. Pharmazeutische Zusammensetzung umfassend eine Verbindung der Formel (I), wie in Anspruch 1 definiert, oder ein therapeutisch annehmbares Additionssalz hievon und einen pharmazeutisch annehmbaren Träger.

3. Kardiotonische pharmazeutische Zusammensetzung, die eine Verbindung der Formel (I)

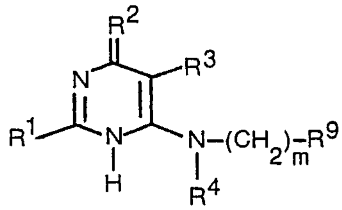

(I)

worin $R^1$ $C_1$—$C_6$-Alkyl, Cyclo($C_3$—$C_6$)alkyl oder Phenyl($C_1$—$C_6$)alkyl bedeutet; $R^2$ Oxo, Thioxo oder Imino darstellt; $R^3$ Cyano, Aminocarbonyl oder Nitro ist; $R^4$ Wasserstoff oder $C_1$—$C_4$-Alkyl bedeutet; $R^9$ Wasserstoff, $C_2$—$C_6$-Alkenyl, 1-Pyridinyl, Cyclo($C_3$—$C_6$)alkyl, 2-, 3- oder 4-Pyridinyl, 2- oder 3-Furanyl, 2- oder 3-Indolyl, 2- oder 3-Thienyl, 5-Imidazolyl, 4-Morpholinyl, Phenyl gegebenenfalls disubstituiert mit $C_1$—$C_6$-Alkoxy oder 4-Thiomorpholinyl darstellt und m eine ganze Zahl von Null bis 2 ist, oder ein therapeutisch annehmbares Salz hievon und ein Mittel, das üblicherweise bei der Behandlung von kongestivem Herzversagen angewandt wird, umfaßt.

4. Kardiontonische pharmazeutische Zusammensetzung, wie in Anspruch 3 beansprucht, worin das genannte Mittel ausgewählt ist aus Isosorbiddinitrat, Captopril, Nifedipin, Hydralazin, Prazosin, Hydrochlorthiazid, Furosemid, Spironolacton, Digitalis und Dobutamin.

5. Verbindung der Formel (I)

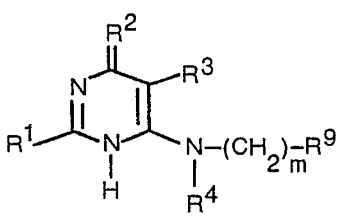

(I)

worin $R^1$ $C_1$—$C_6$-Alkyl, Cyclo($C_3$—$C_6$)alkyl oder Phenyl($C_1$—$C_6$)alkyl bedeutet; $R^2$ Oxo, Thioxo oder Imino darstellt; $R^3$ Cyano, Aminocarbonyl oder Nitro ist; $R^4$ Wasserstoff oder $C_1$—$C_4$-Alkyl bedeutet; $R^9$ $C_2$—$C_6$-Alkenyl, 1-Piperidinyl, Cyclo($C_3$—$C_6$)alkyl, 2-, 3- oder 4-Pyridinyl, 2- oder 3-Furanyl, 2- oder 3-Indolyl, 2- oder 3-Thienyl, 5-Imidazolyl, 4-Moorpholinyl, Phenyl gegebenenfalls disubstituiert mit $C_1$—$C_6$-Alkoxy oder 4-Thiomorpholinyl darstellt und m eine ganze Zahl von Null bis 2 ist, oder ein therapeutisch annehmbares Additionssalz hievon.

6. Verbindung, wie in Anspruch 5 beansprucht, worin $R^1$ $C_1$—$C_6$-Alkyl, Cyclo($C_3$—$C_6$)alkyl oder Benzyl bedeutet; $R^2$ Oxo, Thioxo oder Imino darstellt; $R^3$ Cyano oder Aminocarbonyl ist; $R^4$ Wasserstoff oder $C_1$—$C_4$-Alkyl bedeutet; $R^9$ $C_2$—$C_6$-Alkenyl, Cyclo($C_3$—$C_6$)alkyl, 2-, 3- oder 4-Pyridinyl, 2- oder 3-Furanyl, 2- oder 3-Indolyl, 2- oder 3-Thienyl, 4-Morpholinyl oder Phenyl gegebenenfalls disubstituiert mit $C_1$—$C_6$-

Alkoxy darstellt und m eine ganze Zahl von Null bis 2 ist, oder ein therapeutisch annehmbares Additionssalz hievon.

7. Verbindung, wie in Anspruch 5 beansprucht, worin $R^1$ $C_1$—$C_6$-Alkyl, Cyclo($C_3$—$C_6$)alkyl oder Benzyl bedeutet; $R^2$ Oxo oder Thixo darstellt; $R^3$ Cyano ist; $R^4$ Wasserstoff bedeutet; $R^9$ $C_2$—$C_6$-Alkenyl, 2-, 3- oder 4-Pyridinyl, 2-Furanyl, 3-Idolyl, 3-Thienyl, 4-Morpholinyl oder Phenyl gegebenenfalls disubstituiert mit $C_1$—$C_6$-Alkoxy darstellt und m Null oder 1 ist, oder ein therapeutisch annehmbares Additionssalz hievon.

8. Verbindung, wie in Anspruch 5 beansprucht, die
1,4-Dihydro-2-methyl-4-oxo-6-[(3-pyridinylmethyl)-amino]-5-pyrimidincarbonitril;
1,4-Dihydro-2-methyl-6-[N-methyl-N-(3-pyridinylmethyl)-amino]-4-oxo-5-pyrimidincarbonitril;
1,4-Dihydro-2-methyl-4-oxo-6-[(phenyläthyl)-amino]-5-pyrimidincarbonitril;
1,4-Dihydro-2-methyl-6-[(2-pyridinyläthyl)-amino]-5-pyrimidincarbonitril;
1,4-Dihydro-2-methyl-4-oxo-6-[(4-pyridinylmethyl)-amino]-5-pyrimidincarbonitril;
1,4-Dihydro-2-methyl-4-oxo-6-[(2-pyridinylmethyl)-amino]-5-pyrimidincarbonitril;
1,4-Dihydro-2-methyl-4-oxo-6-[(phenylmethyl)-amino]-5-pyrimidincarbonitril;
1,4-Dihydro-2-methyl-4-oxo-6-[2-(3-pyridinyl)-äthylamino]-5-pyrimidincarbonitril;
1,4-Dihydro-2-methyl-4-oxo-6-[2-(4-pyridinyl)-äthylamino]-5-pyrimidincarbonitril;
1,4-Dihydro-2-methyl-4-oxo-6-[(3-pyridinyl)-amino]-5-pyrimidincarbonitril;
1,4-Dihydro-2-methyl-6-((3-pyridinylmethyl)-amino]-4-tioxo-5-pyrimidincarbonitril;
1,4-Dihydro-2-äthyl-4-oxo-6-[(3-pyridinylmethyl)-amino]-5-pyrimidincarbonitril;
1,4-Dihydro-2-(1-methyläthyl)-4-oxo-6-[(3-pyridinylmethyl)-amino]-5-pyrimidincarbonitril;
2-Cyclopropyl-1,4-dihydro-4-oxo-6-[(3-pyridinylmethyl)-amino]-5-pyrimidincarbonitril;
1,4-Dihydro-2-(1,1-dimethyläthyl)-4-oxo-6-[(3-pyridinylmethyl)-amino]-5-pyrimidincarbonitril;
1,4-Dihydro-4-oxo-2-phenylmethyl-6-[(3-pyridinylmethyl)-amino]-5-pyrimidincarbonitril oder ein pharmazeutisch annehmbares Salz hievon ist.

9. Verbindung, wie in Anspruch 5 beansprucht, die
1,4-Dihydro-6-[(2-furanylmethyl)-amino]-2-methyl-4-oxo-5-pyrimidincarbonitril;
1,4-Dihydro-2-methyl-6-[[(2-(4-morpholinyl)-äthyl]-amino]-4-oxo-5-pyrimidincrbonitril;
1,4-Dihydro-2-methyl-4-oxo-6-[[(2-(3-thienyl)-äthyl]-amino]-5-pyrimidincarbonitril;
1,4-Dihydro-6-[[(2-(3,4-dimethoxyphenyl)-äthyl]-amino]-2-methyl-4-oxo-5-pyrimidincarbonitril;
1,4-Dihydro-6-[[2-(1H-indol-3-yl)-äthyl]-amino]-2-methyl-4-oxo-5-pyrimidincarbonitril;
1,4-Dihydro-2-methyl-4-oxo-6-[(2-propenyl)-amino]-5-pyrimidincarbonitril oder ein pharmazeutisch annehmbares Additionssalz hievon ist.

10. Verfahren zum Herstellen einer Verbindung der Formel (I)

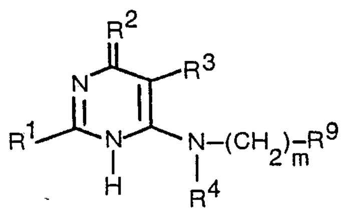

(I)

worin $R^1$ $C_1$—$C_6$-Alkyl, Cyclo($C_3$—$C_6$)alkyl oder Phenyl($C_1$—$C_6$)alkyl bedeutet; $R^2$ Oxo, Thioxo oder Imino darstellt; $R^3$ Cyano, Aminocarbonyl oder Nitro ist; $R^4$ Wasserstoff oder $C_1$—$C_4$-Alkyl bedeutet; $R^9$ Wasserstoff, $C_2$—$C_6$-Alkenyl, 1-Piperidinyl, Cyclo($C_3$—$C_6$)alkyl, 2-, 3- oder 4-Pyridinyl, 2- oder 3- Furanyl, 2- oder 3-Indolyl, 2- oder 3-Thienyl, 5-Imidazolyl, 4-Morpholinyl, Phenyl gegebenenfalls disubstituiert mit $C_1$—$C_6$-Alkoxy oder 4-Thiomorpholinyl darstellt und m eine ganze Zahl von Null bis 2 ist, oder eines therapeutisch annehmbaren Additionssalzes hievon, welches umfaßt:

(a) das Umsetzen eines Pyrimidins (IV)

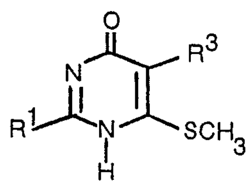

(IV)

worin $R^1$ wie oben definiert ist und $R^3$ Cyano oder Nitro bedeutet, mit einem Amin (V)

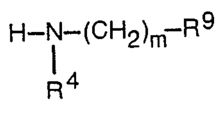

(V)

worin $R^4$, $R^9$ und m wie oben definiert sind, zu der Verbindung der Formel (I), worin $R^2$ Oxo ist, $R^3$ Cyano oder Nitro darstellt und $R^1$, $R^4$, $R^9$ und m wie oben definiert sind, oder

(b) das Umsetzen einer Verbindung der Formel (III)

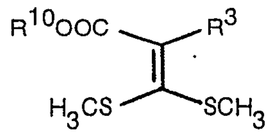

(III)

worin $R^3$ Cyano oder Nitro ist und $R^{10}$ $C_1$—$C_6$-Alkyl bedeutet, mit einem Amin (V)

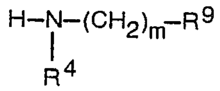

(V)

worin $R^4$, $R^9$ und m wie oben definiert sind, zur Verbindung (VI)

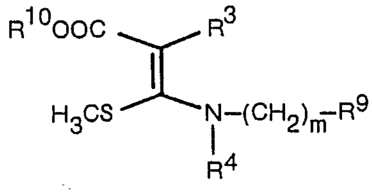

(VI)

worin $R^3$ Cyano oder Nitro ist und $R^4$, $R^9$, $R^{10}$ und m wie oben definiert sind, und Umsetzen der Verbindung (VI) mit einem Amidin (II)

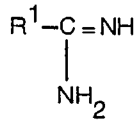

(II)

worin $R^1$ wie oben definiert ist, zu einer Verbindung der Formel (I), worin $R^2$ Oxo ist, $R^3$ Cyano oder Nitro bedeutet und $R^1$, $R^4$, $R^9$ und m wie oben definiert sind,

(c) wenn gewünscht, das überführen einer Verbindung der Formel (I), worin $R^2$ Oxo ist, in eine Verbindung der Formel (I), worin $R^2$ Thioxo ist, durch Umsetzen eines Natriumsalzes einer Verbindung der Formel (I), worin $R^2$ Oxo ist, mit Phosphoroxychlorid, wobei ein 4-Chlorpyrimidin der Formel (VII)

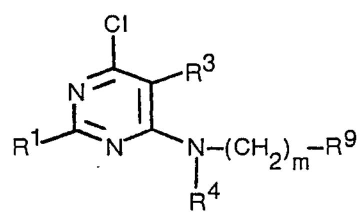

(VII)

erhalten wird, worin $R^3$ Cyano oder Nitro darstellt und $R^1$, $R^4$, $R^9$ und m wie oben definiert sind, und Behandeln des 4-Chlorpyrimidins (VII) mit einer wässerigen Äthanollösung von Kaliumhydroxid und Schwefelwasserstoff, wobei die Verbindung der Formel (I) erhalten wird, worin $R^2$ Thioxo ist, $R^6$ Cyano oder Nitro darstellt und $R^1$, $R^4$, $R^9$ und m wie oben definiert sind,

(d) wenn gewünscht, das Behandeln eines 4-Chlorpyrimidins (VII) mit einer Lösung eines inerten organischen Lösungsmittels, das einen Ammoniaküberschuß enthält, wobei die Verbindung der Formel (I) erhalten wird, worin $R^2$ Imino ist, $R^3$ Cyano oder Nitro bedeutet und $R^1$, $R^4$, $R^9$ und m wie oben definiert sind,

e) wenn gewünscht, das Hydrolysieren einer Verbindung der Formel (I), worin $R^1$ Cyano ist und $R^1$, $R^2$, $R^4$, $R^9$ und m wie oben definiert sind, mit Schwefelsäure, wobei die Verbindung der Formel (I) erhalten wird, worin $R^3$ Aminocarbonyl ist und $R^1$, $R^2$, $R^4$, $R^9$ und m wie oben definiert sind.

11. Verfahren zum Herstellen einer Verbindung der Formel (I) wie in Anspruch 5 beansprucht, welches umfaßt:

(a) das Umsetzen einer Verbindung der Formel (VI)

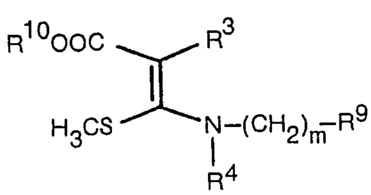

(VI)

worin $R^3$ Cyano oder Nitro bedeutet, $R^4$, $R^9$ und m wie in Anspruch 5 definiert sind und $R^{10}$ $C_1$—$C_6$-Alkyl ist, mit einem Amidin der Formel (II)

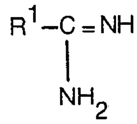

(II)

worin $R^1$ wie in Anspruch 5 definiert ist, zu einer Verbindung der Formel (I), worin $R^2$ Oxo ist, $R^3$ Cyano oder Nitro darstellt und $R^1$, $R^4$, $R^9$ und m wie in Anspruch 5 definiert sind, oder

(b) das Umsetzen einer Verbindung der Formel (VII)

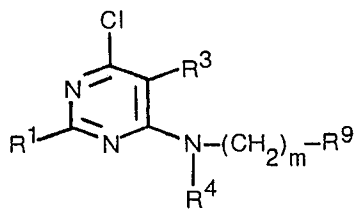

(VII)

worin $R^1$, $R^4$, $R^9$ und m wie in Anspruch 5 definiert sind und $R^3$ Cyano oder Nitro ist, mit einer wässerigen Äthanollösung von Kaliumhydroxid und Schwefelwasserstoff, zu einer Verbindung der Formel (I), worin $R^2$ Thioxo ist, oder

c) das Umsetzen einer Verbindung der Formel (VII), wie oben definiert, mit einer Lösung eines inerten organischen Lösungsmittels, das einen Ammoniaküberscuß enthält, zu einer Verbindung der Formel (I), worin $R^2$ Imino ist, oder

d) das Hydrolysieren einer Verbindung der Formel (I), wie in Anspruch 5 definiert, worin $R^3$ Cyano ist, zu einer Verbindung der Formel (I), worin $R^3$ Aminocarbonyl ist, oder

e) das überführen einer Verbindung der Formel (I), wie in Anspruch 5 definiert, in ein therapeutisch annehmbares Salz hievon.

**Patentansprüche für den Vertragsstaat: AT**

1. Verbindung zum Herstellen einer Verbindung der Formel (I)

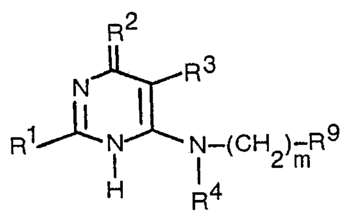

(I)

worin $R^1$ $C_1$—$C_6$-Alkyl, Cyclo($C_3$—$C_6$)alkyl oder Phenyl($C_1$—$C_6$)alkyl bedeutet; $R^2$ Oxo, Thioxo oder Imino darstellt; $R^3$ Cyano, Aminocarbonyl oder Nitro ist; $R^4$ Wasserstoff oder $C_1$—$C_4$-Alkyl bedeutet; $R^9$ Wasserstoff, $C_2$—$C_6$-Alkenyl, 1-Piperidinyl, Cyclo($C_3$—$C_6$)alkyl, 2-, 3- oder 4-Pyridinyl, 2- oder 3-Furanyl, 2- oder 3-Indolyl, 2- oder 3-Thienyl, 5-Imidazolyl, 4-Morpholinyl, Phenyl gegebenenfalls disubstituiert mit $C_1$—$C_6$-Alkoxy oder 4-Thiomorpholinyl darstellt und m eine ganze Zahl von Null bis 2 ist, oder eines therapeutisch annehmbares Additionssalz hievon, welches umfaßt:

(a) das Umsetzen eines Pyrimidins (IV)

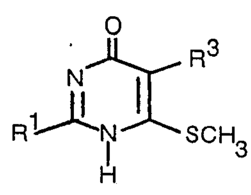

(IV)

worin $R^1$ wie oben definiert ist und $R^3$ Cyano oder Nitro bedeutet, mit einem Amin (V)

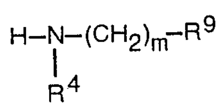

(V)

worin $R^4$, $R^9$ und m wie oben definiert sind, zu der Verbindung der Formel (I), worin $R^2$ Oxo ist, $R^3$ Cyano oder Nitro darstellt und $R^1$, $R^4$, $R^9$ und m wie oben definiert sind, oder

(b) das Umsetzen einer Verbindung der Formel (III)

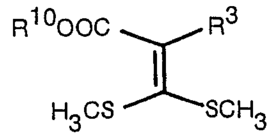

(III)

worin $R^3$ Cyano oder Nitro ist und $R^{10}$ $C_1$—$C_6$-Alkyl bedeutet, mit einem Amin (V)

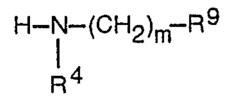

(V)

worin $R^4$, $R^9$ und m wie oben definiert sind, zur Verbindung (VI)

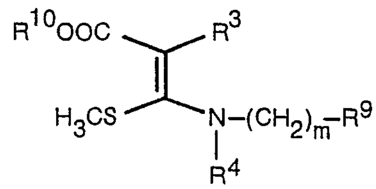

(VI)

worin $R^3$ Cyano oder Nitro ist und $R^4$, $R^9$, $R^{10}$ und m wie oben definiert sind, und Umsetzen der Verbindung (VI) mit einem Amidin (II)

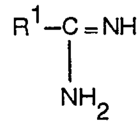

(II)

worin $R^1$ wie oben definiert ist, zu einer Verbindung der Formel (I), worin $R^2$ Oxo ist,$R^3$ Cyano oder Nitro bedeutet und $R^1$, $R^4$, $R^9$ und m wie oben definiert sind,

(c) wenn gewünscht, das überführen einer Verbindung der Formel (I), worin $R^2$ Oxo ist, in eine Verbindung der Formel (I), worin $R^2$ Thioxo ist, durch Umsetzen eines Natriumsalzes einer Verbindung der Formel (I), worin $R^2$ Oxo ist, mit Phosphoroxychlorid, wobei ein 4-Chlorpyrimidin der Formel (VII)

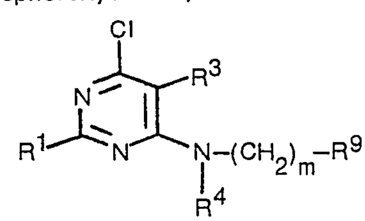

(VII)

erhalten wird, worin $R^3$ Cyano oder Nitro darstellt und $R^1$, $R^4$, $R^9$ und m wie oben definiert sind, und Behandeln des 4-Chlorpyrimidins (VII) mit einer wässerigen Äthanollösung von Kaliumhydroxid und Schwefelwasserstoff, wobei die Verbindung der Formel (I) erhalten wird, worin $R^2$ Thioxo ist, $R^3$ Cyano oder Nitro darstellt und $R^1$, $R^4$, $R^9$ und m wie oben definiert sind,

(d) wenn gewünscht, das Behandeln eines 4-Chlorpyrimidins (VII) mit einer Lösung eines inerten organischen Lösungsmittels, das einen Ammoniaküberschuß enthält, wobei die Verbindung der Formel (I) erhalten wird, worin $R^2$ Imino ist, $R^3$ Cyano oder Nitro bedeutet und $R^1$, $R^4$, $R^9$ und m wie oben definiert sind,

e) wenn gewünscht, das Hydrolysieren einer Verbindung der Formel (I), worin $R^3$ Cyano ist und $R^1$, $R^2$, $R^4$, $R^9$ und m wie oben definiert sind, mit Schwefelsäure, wobei die Verbindung der Formel (I) erhalten wird, worin $R^3$ Aminocarbonyl ist und $R^1$, $R^2$, $R^4$, $R^9$ und m wie oben definiert sind.

2. Verfahren zum Herstellen einer Verbindung der Formel (I)

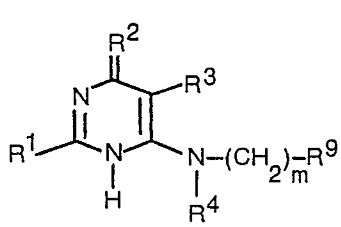

(I)

worin $R^1$ $C_1$—$C_6$-Alkyl, Cyclo($C_3$—$C_6$)alkyl oder Phenyl($C_1$—$C_6$)alkyl bedeutet; $R^2$ Oxo, Thioxo oder Imino

darstellt; $R^3$ Cyano, Aminocarbonyl oder Nitro ist; $R^4$ Wasserstoff oder $C_1$—$C_4$-Alkyl bedeutet; $R^9$ $C_2$—$C_6$-Alkenyl, 1-Piperidinyl, Cyclo($C_3$—$C_6$)alkyl, 2-, 3- oder 4-Pyridinyl, 2- oder 3-Furanyl, 2- oder 3-Indolyl, 2- oder 3-Thienyl, 5-Imidazolyl, 4-Morpholinyl, Phenyl gegebenenfalls disubstituiert mit $C_1$—$C_6$-Alkoxy oder 4-Thiomorpholinyl darstellt und m eine ganze Zahl von Null bis 2 ist, oder ein therapeutisch annehmbaren Additionssalzes hievon, welches umfaßt:

(a) das Umsetzen einer Verbindung der Formel (VI)

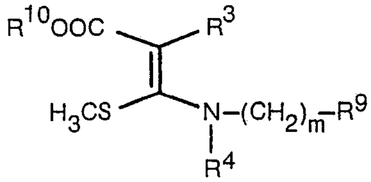

(VI)

worin $R^3$ Cyano oder Nitro bedeutet, $R^4$, $R^9$ und m wie oben definiert sind und $R^{10}$ $C_1$—$C_6$-Alkyl ist, mit einem Amidin der Formel (II)

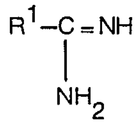

(II)

worin $R^1$ wie oben definiert ist, zu einer Verbindung der Formel (I), worin $R^2$ Oxo ist, $R^3$ Cyano oder Nitro darstellt und $R^1$ $R^4$, $R^9$ und m wie oben definiert sind, oder

(b) das Umsetzen einer Verbindung der Formel (VII)

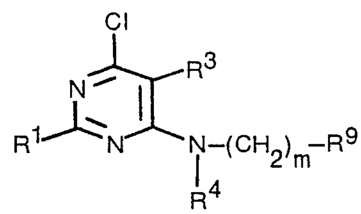

(VII)

worin $R^1$, $R^4$, $R^9$ und m wie oben definiert sind und $R^3$ Cyano oder Nitro ist, mit einer wässerigen Äthanollösung von Kaliumhydroxid und Schwefelwasserstoff, zu einer Verbindung der Formel (I), worin $R^2$ Thioxo ist, oder

c) das Umsetzen einer Verbindung der Formel (VII), wie oben definiert, mit einer Lösung eines inerten organischen Lösungsmittels, das einen Ammoniaküberschuß enthält, zu einer Verbindung der Formel (I), worin $R^2$ Imino ist, oder

d) das Hydrolysieren einer Verbindung der Formel (I), wie in Anspruch 5 definiert, worin $R^3$ Cyano ist, zu einer Verbindung der Formel (I), worin $R^3$ Aminocarbonyl ist, oder

e) das überführen einer Verbindung der Formel (I), wie oben definiert, in ein therapeutisch annehmbares Salz hievon.

3. Verfahren, wie in Anspruch 1 beansprucht, zum Herstellen einer Verbindung der Formel (I), worin $R^1$ $C_1$—$C_6$-Alkyl, Cyclo($C_3$—$C_6$)alkyl oder Benzyl bedeutet; $R^2$ Oxo, Thioxo oder Imino darstellt; $R^3$ Cyano oder Aminocarbonyl ist; $R^4$ Wassrstoff oder $C_1$—$C_4$-Alkyl bedeutet; $R^9$ $C_2$—$C_6$-Alkenyl, Cyclo($C_3$—$C_6$)alkyl, 2-, 3- oder 4-Pyridinyl, 2- oder 3-Furanyl, 2- oder 3-Indolyl, 2- oder 3-Thienyl, 4-Morpholinyl oder Phenyl gegebenenfalls disubstituiert mit $C_1$—$C_6$-Alkoxy darstellt und m eine ganze Zahl von Null bis 2 ist, oder eines therapeutisch annehmbaren Additionssalzes hievon.

4. Verfahren, wie in Anspruch 1 beansprucht, zum Herstellen einer Verbindung der Formel (I), worin $R^1$ nied.Alkyl, Cyclo(nied.)alkyl oder Benzyl ist, $R^2$ Oxo oder Thioxo bedeutet, $R^3$ Cyano ist, $R^4$ Wasserstoff darstellt, $R^9$ $C_2$—$C_6$- Alkenyl, 2-, 3- oder 4-Pyridinyl, 2-Furanyl, 3-Indolyl, 3-Thienyl, 4-Morpholinyl oder Phenyl gegebenenfalls disubstituiert mit $C_1$—$C_6$-Alkoxy darstellt und m Null oder 1 ist, oder eines therapeutisch annehmbaren Additionssalzes hievon.

5. Verfahren, wie in Anspruch 1 beansprucht, worin die hergestellte Verbindung der Formel

1,4-Dihydro-2-methyl-4-oxo-6-[(3-pyridinylmethyl)-amino]-5-pyrimidincarbonitril;
1,4-Dihydro-2-methyl-6-[N-methyl-N-(3-pyridinylmethyl)-amino]-4-oxo-5-pyrimidincarbonitril;
1,4-Dihydro-2-methyl-4-oxo-6-[(phenyläthyl)-amino]-5-pyrimidincarbonitril;
1,4-Dihydro-2-methyl-4-oxo-6-[(2-pyridinyläthyl)-amino]-5-pyrimidincarbonitril;
1,4-Dihydro-2-methyl-4-oxo-6-[(4-pyridinylmethyl)-amino]-5-pyrimidincarbonitril;
1,4-Dihydro-2-methyl-4-oxo-6-[(2-pyridinylmethyl)-amino]-5-pyrimidincarbonitril;
1,4-Dihydro-2-methyl-4-oxo-6-[(phenylmethyl)-amino]-5-pyrimidincarbonitril;
1,4-Dihydro-2-methyl-4-oxo-6-[2-(3-pyridinyl)-äthylamino]-5-pyrimidincarbonitril;
1,4-Dihydro-2-methyl-4-oxo-6-[2-(4-pyridinyl)-äthylamino]-5-pyrimidincarbonitril;
1,4-Dihydro-2-methyl-4-oxo-6-[(3-pyridinyl)-amino]-5-pyrimidincarbonitril;

1,4-Dihydro-2-methyl-6-((3-pyridinylmethyl)-amino]-4-thioxo-5-pyrimidincarbonitril;
1,4-Dihydro-2-äthyl-4-oxo-6-[(3-pyridinylmethyl)-amino]-5-pyrimidincarbonitril;
1,4-Dihydro-2-(1-methyläthyl)-4-oxo-6-[(3-pyridinylmethyl)-amino]-5-pyrimidincarbonitril;
2-Cyclopropyl-1,4-dihydro-4-oxo-6-[(3-pyridinylmethyl)-amino]-5-pyrimidincarbonitril;
1,4-Dihydro-2-(1,1-dimethyläthyl)-4-oxo-6-(3-pyridinylmethyl)-amino]-5-pyrimidincarbonitril;
1,4-Dihydro-4-oxo-2-phenylmethyl-6-[(3-pyridinylmethyl)-amino]-5-pyrimidincarbonitril oder ein pharmazeutisch annehmbares Salz hievon ist.

6. Verfahren, wie in Anspruch 1 beanspurcht, worin die hergestellte Vrbindung der Formel (I)
1,4-Dihydro-6-[(2-furanylmethyl)-amino]-2-methyl-4-oxo-5-pyrimidincarbonitril;
1,4-Dihydro-2-methyl-6-[[(2-(4-morpholinyl)-äthyl]-amino]-4-oxo-5-pyrimidincrbonitril;
1,4-Dihydro-2-methyl-4-oxo-6-[[(2-(3-thienyl)-äthyl]-amino]-5-pyrimidincarbonitril;
1,4-Dihydro-6-[[(2-(3,4-dimethoxyphenyl)-äthyl]-amino]-2-methyl-4-oxo-5-pyrimidincarbonitril;
1,4-Dihydro-6-[[2-(1H-indol-3-yl)-äthyl]-amino]-2-methyl-4-oxo-5-pyrimidincarbonitril;
1,4-Dihydro-2-methyl-4-oxo-6-[(2-propenyl)-amino]-5-pyrimidincarbonitril oder ein pharmazeutisch annehmbares Additionssalz hievon ist.

7. Verfahren zum Herstellen einer pharmazeutischen Zusammensetzung, welches darin besteht, daß man eine Verbindung der Formel (I), wie in Anspruch 1 definiert, oder ein therapeutisch annehmbares Salz hievon in eine für therapeutische Verabreichung geeignete For bringt.

8. Verfahren, wie in Anspruch 7 beansprucht, worin die Zusammensetzung auch ein Mittel aufweist, das üblicherweise bei der Behandlung von kongestivem Herzversagen verwendet wird.

9. Verfahren, wie in Anspruch 8 beansprucht, worin dieses Mittel ausgewähtl ist aus Isosorbiddinitrat, Captopril, Nifedipin, Hydralazin, Prazosin, Hydrochlorthiazid, Furosemid, Spironolacton, Digitalis und Dobutamin.

**Revendications pour les Etats contractants: BE CH DE FR GB IT LI LU NL SE**

1. Composé de formule I

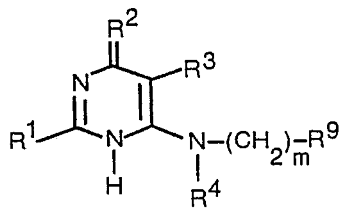

(I)

dans laquelle $R^1$ représente un groupe alkyle en $C_1$ à $C_6$, cyclo-(alkyle en $C_3$ à $C_6$) ou phényl-(alkyle en $C_1$ à $C_6$); $R^2$ représente un groupe oxo, thioxo ou imino; $R^3$ représente un groupe cyano, aminocarbonyle ou nitro; $R^4$ représente l'hydrogène ou un groupe alkyle en $C_1$ à $C_4$; $R^9$ représente l'hydrogène, un groupe alcényle en $C_2$ à $C_6$; 1-pipéridinyle, cyclo-(alkyle en $C_3$ à $C_6$), 2,3- ou 4-pyridinyle, 2- ou 3 furannyle, 2- ou 3-indolyle, 2- ou 3-thiényle, 5-imidazolyle, 4-morpholinyle, phényle facultativement disubstitué avec un groupe alkoxy en $C_1$ à $C_6$, ou 4-thiomorpholinyle, et *m* est un nombre entier de 0 à 2; ou un de ses sels d'addition thérapeutiquement acceptables, destiné à être utilisé comme substance pharmaceutique.

2. Composition pharmaceutique comprenant un composé de formule I suivant la revendication 1 ou un de ses sels d'addition thérapeutiquement acceptables et un support pharmaceutiquement acceptable.

3. Composition pharmaceutique cardiotonique, qui comprend un composé de formule I

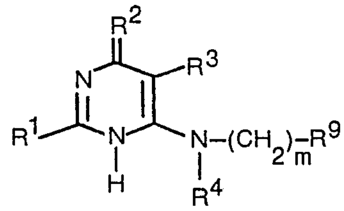

(I)

dans laquelle $R^1$ représente un groupe alkyle en $C_1$ à $C_6$, cyclo-(alkyle en $C_3$ à $C_6$) ou phényl-(alkyle en $C_1$ à $C_6$); $R^2$ représente un groupe oxo, thioxo ou imino; $R^3$ représente un groupe cyano, aminocarbonyle ou nitro; $R^4$ représente l'hydrogène ou un groupe alkyle en $C_1$ à $C_4$; $R^9$ représente l'hydrogène, un groupe alcényle en $C_2$ à $C_6$; 1-pipéridinyle, cyclo-(alkyle en $C_3$ à $C_6$), 2,3- ou 4-pyridinyle, 2- ou 3 furannyle, 2- ou 3-indolyle, 2- ou 3-thiényle, 5-imidazolyle, 4-morpholinyle, phényle facultativement disubstitué avec un groupe alkoxy en $C_1$ à $C_6$, 4-thiomorpholinyle, et *m* est un nombre entier de 0 à 2; ou un de ses sels thérapeutiquement acceptables, et un agent couramment utilisé dans le traitement de l'insuffisance cardiaque congestive.

4. Composition pharmaceutique cardiotonique suivant la revendication 3, dans laquelle l'agent est choisi entre l'isosorbide dinitrate, le captopril, la nifédipine, l'hydralazine, la prazosine, l'hydrochlorothiazide, le furosémide, la spironolactone, la digitaline et la dobutamine.

5. Composé de formule I

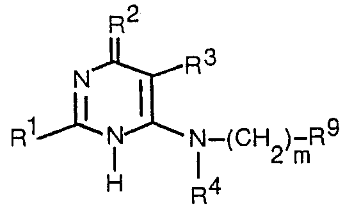

dans laquelle $R^1$ représente un groupe alkyle en $C_1$ à $C_6$, cyclo-(alkyle en $C_3$ à $C_6$) ou phényl-(alkyle en $C_1$ à $C_6$); $R^2$ représente un groupe oxo, thioxo ou imino; $R^3$ représente un groupe cyano, aminocarbonyle ou nitro; $R^4$ représente l'hydrogène ou un groupe alkyle en $C_1$ à $C_4$; $R^9$ représente un groupe alcényle en $C_2$ à $C_6$; 1-pipéridinyle, cyclo-(alkyle en $C_3$ à $C_6$), 2,3- ou 4-pyridinyle, 2- ou 3 furannyle, 2- ou 3-indolyle, 2- ou 3-thiényle, 5-imidazolyle, 4-morpholinyle, phényle facultativement disubstitué avec un groupe alkoxy en $C_1$ à $C_6$, ou 4-thiomorpholinyle, et *m* est un nombre entier de 0 à 2; ou un de ses sels d'addition thérapeutiquement acceptables.

6. Composé suivant la revendication 5, dans laquel $R^1$ représente un groupe alkyle en $C_1$ à $C_6$, cyclo-(alkyle en $C_3$ à $C_6$) ou benzyle; $R^2$ représente un groupe oxo, thioxo ou imino; $R^3$ représente un groupe cyano ou aminocarbonyle; $R^4$ représente l'hydrogène ou un groupe alkyle en $C_1$ à $C_4$; $R^9$ représente un groupe alcényle en $C_2$ à $C_6$; cyclo-(alkyle en $C_3$ à $C_6$), 2,3- ou 4-pyridinyle, 2- ou 3 furannyle, 2- ou 3-indolyle, 2- ou 3-thiényle, 4-morpholinyle, phényle facultativement disubstitué avec un groupe alkoxy en $C_1$ à $C_6$, et *m* est un nombre entier de 0 à 2; ou un de ses sels thérapeutiquement acceptables.

7. Composé suivant la revendication 5, dans laquel $R^1$ représente un groupe alkyle en $C_1$ à $C_6$, cyclo-(alkyle en $C_3$ à $C_6$) ou benzyl; $R^2$ représente un groupe oxo, thioxo, $R^3$ représente un groupe cyano; $R^4$ représente l'hydrogène; $R^9$ représente un groupe alcényle en $C_1$ à $C_6$, 2,3- ou 4-pyridinyle, 2-furannyle, 3-indolyle, 3-thiényle, 4-morpholinyle, phényle facultativement disubstitué avec un groupe alkoxy en $C_1$ à $C_6$; et *m* a la valeur 0 ou 1; ou un de sels d'addition thérapeutiquement acceptables.

8. Composé suivant la revendication 5, qui est le
1,4-dihydro-2-méthyl-4-oxo-6-[(3-pyridinylméthyl)amino]-5-pyrimidinecarbonitrile;
le 1,4-dihydro-2-méthyl-6-[N-méthyl-N-(3-pyridinylméthyl)amino]-4-oxo-5-pyrimidinecarbonitrile;
le 1,4-dihydro-2-méthyl-4-oxo-6-[(phényléthyl)amino]-5-pyrimidinecarbonitrile;
le 1,4-dihydro-2-méthyl-4-oxo-6-[(2-pyridinyléthyl)amino]-5-pyrimidinecarbonitrile;
le 1,4-dihydro-2-méthyl-4-oxo-6-[(4-pyridinylméthyl)amino]-5-pyrimidinecarbonitrile;
le 1,4-dihydro-2-méthyl-4-oxo-6-[(2-pyridinylméthyl)amino]-5-pyrimidinecarbonitrile;
le 1,4-dihydro-2-méthyl-4-oxo-6-[(phénylméthyl)amino]-5-pyrimidinecarbonitrile;
le 1,4-dihydro-2-méthyl-4-oxo-6-[2-(3-pyridinyl)éthylamino]-5-pyrimidinecarbonitrile;
le 1,4-dihydro-2-méthyl-4-oxo-6-[2-(4-pyridinyl)éthylamino]-5-pyrimidinecarbonitrile;
le 1,4-dihydro-2-méthyl-4-oxo-6-[(3-pyridinyl)amino]-5-pyrimidinecarbonitrile;
le 1,4-dihydro-2-méthyl-6-[(3-pyridinylméthyl)amino]-4-thioxo-5-pyrimidinecarbonitrile;
le 1,4-dihydro-2-éthyl-4-oxo-6-[(3-pyridinylméthyl)amino]-5-pyrimidinecarbonitrile;
le 1,4-dihydro-2-méthylethyl-4-oxo-6-[(3-pyridinylméthyl)amino]-5-pyrimidinecarbonitrile;
le 2-cyclopropyl-1,4-dihydro-4-oxo-6-[(3-pyridinylméthyl)amino]-5-pyrimidinecarbonitrile;
le 1,4-dihydro-2-(1,1-diméthyléthyl)-4-oxo-6-[(3-pyridinylméthyl)amino]-5-pyrimidinecarbonitrile; le 1,4-dihydro-4-oxo-2-phénylméthyl-6-[(3-pyridinylméthyl)amino]-5-pyrimidinecarbonitrile; ou un de ses sels pharmaceutiquement acceptables.

9. Composé suivant la revendication 5, qui est le
1,4-dihydro-6-[(2-furannylméthyl)amino)]-2-méthyl-4-oxo-5-pyrimidinecarbonitrile;
le 1,4-dihydro-2-méthyl-6-[[2-(4-morpholinyl)éthyl)amino]-4-oxo-5-pyrimidinecarbonitrile;
le 1,4-dihydro-2-méthyl-4-oxo-6-[[2-(3-thiényl)-éthyl]amino]-5-pyrimidinecarbonitrile;
le 1,4-dihydro-6-[[2-(3,4-diméthoxyphényl)éthyl]amino]-2-méthyl-4-oxo-5-pyrimidinecarbonitrile;
le 1,4-dihydro-6-[[2-(1H-indole-3-yl)éthyl]amino]-2-méthyl-4-oxo-5-pyrimidinecarbonitrile;
le 1,4-dihydro-2-méthyl-4-oxo-6-[(2-propényl)amino]-5-pyrimidinecarbonitrile; ou un de ses sels d'addition pharmaceutiquement acceptables.

10. Procédé de préparation d'un composé de formule I

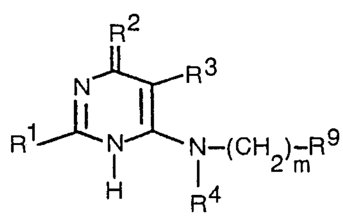
(I)

dans laquelle $R^1$ représente un groupe alkyle en $C_1$ à $C_6$, cyclo-(alkyle en $C_3$ à $C_6$) ou phényl-(alkyle en $C_1$ à $C_6$); $R^2$ représente un groupe oxo, thioxo ou imino; $R^3$ représente un groupe cyano, aminocarbonyle ou nitro; chaque substituant $R^4$ représente, indépendamment, l'hydrogène ou un grupe alkyle en $C_1$ à $C_4$; $R^9$ représente l'hydrogène, un groupe alcényle en $C_2$ à $C_6$, 1-pipéridinyle, cyclo-(alkyle en $C_3$ à $C_6$), 2,3- ou 4-pyridinyle, 2- ou 3-furannyle, 2- ou 3-indolyle, 2- ou 3-thiényle, 5-imidazolyle, 4-morpholinyle, phényle facultativement disubstitué avec un groupe alkoxy en $C_1$ à $C_6$, ou 4-thiomorpholinyle; et *m* est un nombre entier de 0 à 2; ou un de ses sels d'addition thérapeutiquement acceptables, qui consiste:

(a) à faire réagir un pyrimidine de formule IV

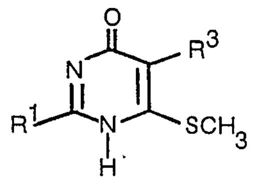

(IV)

dans laquelle $R^1$ répond à la définition précité et $R^3$ représente un groupe cyano ou nitro, avec une amine de formule V

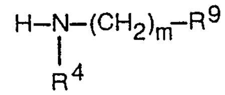

(V)

dans laquelle $R^4$, $R^9$ et *m* répondent aux définitions précitées, pour obtenir le composé correspondant à la formule I, dans laquelle $R^2$ représente un groupe oxo et $R^3$ représente un groupe cyano ou nitro, et $R^1$, $R^4$, $R^9$ et *m* répondent aux définitions précitées, ou bien

(b) à faire réagir un composé de formule III

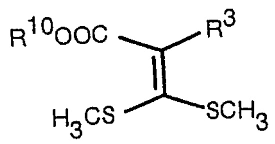

(III)

dans laquelle $R^3$ représente un groupe cyano ou nitro et $R^{10}$ représente un groupe alkyle en $C_1$ à $C_6$, avec une amine de formule V

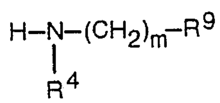

(V)

dans laquelle $R^4$, $R^9$ et *m* répondent aux définitions précitées, pour obtenir le composé de formule VI

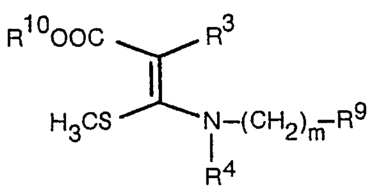

(VI)

dans laquelle $R^3$ représente un groupe cyano ou nitro, et $R^4$, $R^9$, $R^{10}$ et *m* répondent aux définitions précitées, et à faire réagir le composé de formule VI avec une amidine de formule II

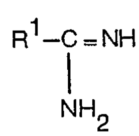

(II)

dans laquelle $R^1$ répond à la définition précitée, pour donner un composé correspondant à la formule I, dans laquelle $R^2$ représente un groupe oxo, $R^3$ représente un groupe cyano ou nitro et $R^1$, $R^4$, $R^9$ et *m* répondent aux définitions précitées,

(c) le cas échéant, à transformer un composé de formule I dans laquelle $R^2$ représenté un groupe oxo en un composé correspondant à la formule I dans laquelle $R^2$ représente un groupe thioxo par réaction d'un sel de sodium d'un composé de formule I dans laquelle $R^2$ représente un groupe oxo avec l'oxychlorure de phosphore pour obtenir une 4-chloropyrimidine de formule VII

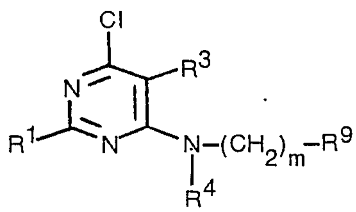

(VII)

dans laquelle $R^3$ représente un groupe cyano ou nitro et $R^1$, $R^4$, $R^9$ et *m* répondent aux définitions précitées, et traitement de la 4-chloropyrimidine de formule VII avec une solution éthanolique aqueuse d'hydroxyde de potassium et d'hydrogène sulfuré pour obtenir le composé correspondant à la formule I dans laquelle $R^2$ représente un groupe thioxo, $R^3$ représente un groupe cyano ou nitro et $R^1$, $R^4$, $R^9$ et *m* répondent aux définitions précitées,

(d) le cas échéant, à traiter une 4-chloropyrimidine de formule VII avec une solution d'un solvant organique inerte contenant un excès d'ammoniac pour obtenir le composé correspondant à la formule I dans laquelle $R^2$ représente un groupe imino, $R^3$ repréente un groupe cyano ou nitro, et $R^1$, $R^4$, $R^9$ et *m* répondent aux définitions précitées,

(e) le cas échéant, à hydrolyser un composé de formule dans laquelle $R^3$ représente un groupe cyano; et $R^1$, $R^2$, $R^4$, $R^9$ et *m* répondent aux définitions précitées, avec l'acide sulfurique pour obtenir le composé correspondant à la formule I dans laquelle $R^3$ représente un groupe aminocarbonyle; et $R^1$,$R^2$, $R^4$, $R^9$ et *m* répondent aux définitions précitées.

11. Procédé de préparation d'un composé de formule I suivant la revendication 5, qui consiste:

a) à faire réagir un composé de formule VI

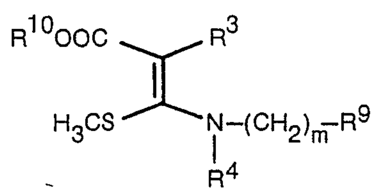

(VI)

dans laquelle $R^3$ représente un groupe cyano ou nitro, et $R^4$,

$R^9$ et *m* répondent aux définitions suivant la revendication 5, et $R^{10}$ représente un groupe alkyle en $C_1$ à $C_6$, avec une amidine de formule II

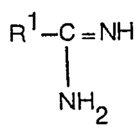

(II)

dans laquelle $R^1$répond à la définition suivant la revendication 5, pour donner un composé de formule I dans laquelle $R^2$ représente un groupe oxo, $R^3$ représente un groupe cyano ou nitro et $R^1$, $R^4$, $R^9$ et *m* répondent aux définitions suivant la revendication 5, ou bien

b) à fair réagir un composé de formule VII

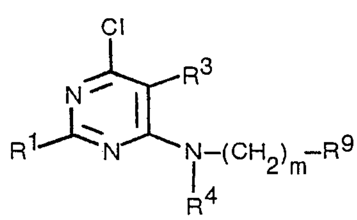

(VII)

dans laquelle $R^1$, $R^4$, $R^8$ et *m* répondent aux définitions suivant la revendication 5 et $R^3$ représente un groupe cyano ou nitro, avec une solution éthanolique aqueuse d'hydroxyde de potassium et d'hydrogène sulfuré, pour donner un composé correspondant de formule I dans laquelle X représente un groupe thioxo, ou bien

c) à faire réagir un composé de formule VII répondant à la définition précité avec une solution d'un solvant organique inerte contenant un excès d'ammoniac pour donner un composé correspondant de formule I dans laquelle $R^2$ représente un groupe imino, ou bien

d) à hydrolyser un composé de formule I répondant à la définition suivant la revendication 5 dans laquelle $R^3$ représente un groupe cyano pour donner un composé de formule I dans laquelle $R^3$ représente un groupe aminocarbonyle, ou bien

e) à transformer un composé de formule I répondant à la définition suivant la revendication 5 en un de ses sels thérapeutiquement acceptables.

**Revendications pour l'Etat contractant: AT**

1. Procédé de préparation d'un composé de formule I

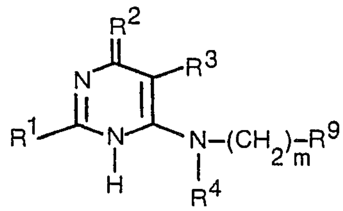

(I)

dans laquelle $R^1$ représente un groupe alkyle en $C_1$ à $C_6$, cyclo-(alkyle en $C_3$ à $C_6$) ou phényl-(alkyle en $C_1$ à $C_6$); $R^2$ représente un groupe oxo, thioxo ou imino; $R^3$ représente un groupe cyano, aminocarbonyle ou nitro; $R^4$ représente l'hydrogène ou un groupe alkyle en $C_1$ à $C_4$; $R^9$ représente l'hydrogène, un groupe alcényle en $C_2$ à $C_6$; 1-pipéridinyle, cyclo-(alkyle en $C_3$ à $C_6$), 2,3- ou 4-pyridinyle, 2- ou 3 furannyle, 2- ou 3-indolyle, 2- ou 3-thiényle, 5-imidazolyle, 4-morpholinyle, phényle facultativement disubstitué avec un groupe alkoxy en $C_1$ à $C_6$, ou 4-thiomorpholinyle, et *m* est un nombre entier de 0 à 2; ou d'un de ses sels d'addition thérapeutiquement acceptables, qui consiste:

(a) à faire réagir une pyrimidine de formule IV

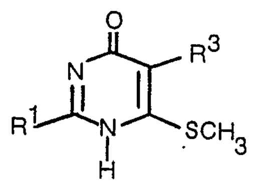

(IV)

dans laquelle $R^1$ répond à la définition précitée $R_3$ représente un grupe cyano ou nitro, avec une amine de formule V

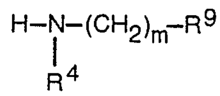

(V)

dans laquelle $R^4$, $R^9$ et *m* répondent aux définitions précitées, pour obtenir le composé correspondant à la formule I, dans laquelle $R^2$ représente un groupe oxo et $R^3$ représente un groupe cyano ou nitro, et $R^1$, $R^4$, $R^9$ et *m* répondent aux définitions précitées, ou bien

(b) à faire réagir un composé de formule III

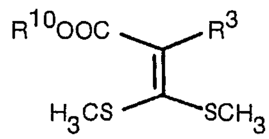

(III)

dans laquelle $R^3$ représente un groupe cyano ou nitro et $R^{10}$ représente un groupe alkyle en $C_1$ à $C_6$, avec une amine de formule V

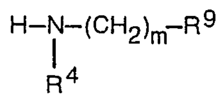

(V)

dans laquelle $R^4$, $R^9$ et *m* répondent aux définitions précitées, pour obtenir le composé de formule VI

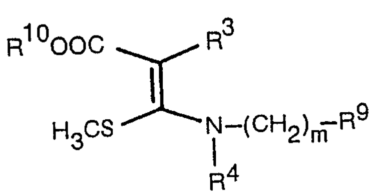

(VI)

dans laquelle $R^3$ représente un groupe cyano ou nitro, et $R^4$, $R^9$, $R^{10}$ et *m* répondent aux définitions précitées, et à faire réagir le composé de formule VI avec une amidine de formule II

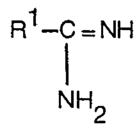

(II)

dans laquelle $R^1$ répond à la définition précitée, pour donner un composé correspondant à la formule I, dans laquelle $R^2$ représente un groupe oxo, $R^3$ représente un groupe cyano ou nitro et $R^1$, $R^4$, $R^9$ et *m* répondent aux définitions précitées,

(c) le cas échéant, à transformer un composé de formule I dans laquelle $R^2$ représente un grupe oxo en un composé correspondant à la formule I dans laquelle $R^2$ représente un groupe thioxo par réaction d'un sel de sodium d'un composé de formule I dans laquelle $R^2$ représente un groupe oxo avec l'oxychlorure de phosphore pour obtenir une 4-chloropyrimidine de formule VII

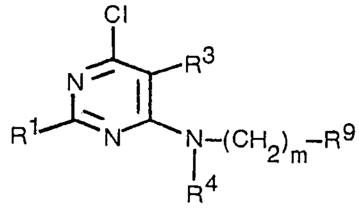

(VII)

dans laquelle $R^3$ représente un groupe cyano ou nitro et $R^1$, $R^4$, $R^9$ et *m* répondent aux définitions précitées, et à traiter la 4-chloropyrimidine de formule VII avec une solution éthanolique aqueuse d'hydroxyde de potassium et d'hydrogène sulfuré pour obtenir le composé correspondant à la formule I dans laquelle $R^2$ représente un groupe thioxo, $R^3$ représente un groupe cyano ou nitro et $R^1$, $R^4$, $R^9$ et *m* répondent aux définitions précitées,

(d) le cas échéant, à traiter une 4-chloropyrimidine de formule VII avec une solution d'un solvant organique inerte contenant un excès d'ammoniac pour obtenir le composé correspondant à la formule I dans laquelle $R^2$ représente un groupe imino, $R^3$ représente un groupe cyano ou nitro, et $R^1$, $R^4$, $R^9$ et *m* répondent aux définitions précitées,

(e) le cas échéant, à hydrolyser un composé de formule dans laquelle $R^3$ représente un groupe cyano; et $R^1$, $R^2$, $R^4$, $R^9$ et *m* répondent aux définitions précitées, avec l'acide sulfurique pour obtenir le composé correspondant à la formule I dans laquelle $R^3$ représente un groupe aminocarbonyle; et $R^1$,$R^2$, $R^4$, $R^9$ et *m* et *n* répondent aux définitions précitées.

2. Procédé de préparation d'un composé de formule I

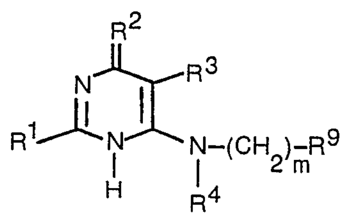

(I)

dans laquelle $R^1$ représente un groupe alkyle en $C_1$ à $C_6$, cyclo-(alkyle en $C_3$ à $C_6$) ou phényl-(alkyle en $C_1$ à $C_6$); $R^2$ représente un groupe oxo, thioxo ou imino; $R^3$ représente un groupe cyano, aminocarbonyle ou nitro; $R^4$ représente, l'hydrogène ou un groupe alkyle en $C_1$ à $C_6$; $R^9$ représente un groupe alcényle en $C_2$ à $C_6$, 1-pipéridinyle, cyclo-(alkyle en $C_3$ à $C_6$), 2,3- ou 4-pyridinyle, 2- ou 3-furannyle, 2- ou 3-indolyle, 2- ou 3-thiényle, 5-imidazolyle, 4-morpholinyle, phényle facultativement disubstitué avec un groupe alkoxy en $C_1$ à $C_6$, 4-thiomorpholinyle; et *m* est un nombre entier de 0 à 2; ou d'un de ses sels d'addition thérapeutiquement acceptables, qui consiste:

(a) à faire réagir un composé de formule IV

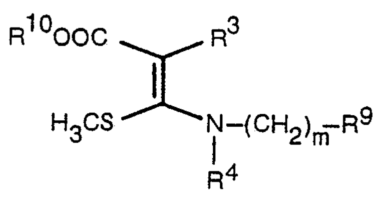

(VI)

dans laquelle $R^3$ représente un groupe cyano ou nitro et $R^4$, $R^9$ et *m* répondent aux définitions précitées et $R^{10}$ est un groupe alkyle en $C_1$ à $C_6$, avec une amidine de formule II

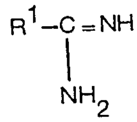

(II)

dans laquelle $R^1$ répond à la définition précitée, pour donner un composé de formule I, dans laquelle $R^2$ représente un groupe oxo, $R^3$ représente un groupe cyano ou nitro et $R^1$, $R^4$, $R^9$ et *m* répondent aux définitions précitées, ou bien

b) à faire réagir un composé de formule VII

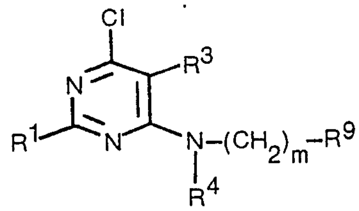

(VII)

dans laquelle $R^1$, $R^4$, $R^9$ et *m* répondent aux définitions précitées et $R^3$ représente un groupe cyano ou nitro, avec une solution éthanolique aqueuse d'hydroxyde de potassium et d'hydrogène sulfuré pour donner un composé correspondant de formule I dans laquelle X représente un groupe thioxo, ou bien

(c) à faire réagir un composé de formule VII répondant à la définition précitée avec une solution formée d'un solvant organique inerte contenant un excès d'ammoniac pour donner un composé correspondant de formule I dans laquelle $R^2$ représente un groupe imino, ou bien

d) à hydrolyser un composé de formule I répondant à la définition précitée, dans laquelle $R^3$ représente un groupe cyano, pour donner un composé de formule I dans laquelle $R^3$ représente un groupe aminocarbonyle, ou bien

e) à transformer un composé de formule I répondant à la définition précitée en un de ses sels thérapeutiquement acceptables.

3. Procédé suivant la revendication 1, pour la préparation d'un composé de formule I dans laquelle $R^1$ représente un groupe alkyle en $C_1$ à $C_6$, cyclo-(alkyle en $C_3$ à $C_6$) ou benzyle; $R^2$ représente un groupe oxo, thioxo ou imino; $R^3$ représente un groupe cyano ou aminocarbonyle; $R^4$ représente l'hydrogène ou un groupe alkyle en $C_1$ à $C_4$; $R^9$ représente un groupe alcényle en $C_2$ à $C_6$, cyclo-(alkyle en $C_3$ à $C_6$), 2,3- ou 4-pyridinyle, 2- ou 3-furannyle, 2- ou 3-indoyle, 2- ou 3-thiényle, 4-morpholinyle, phényle facultativement disubstitué avec un groupe alkoxy en $C_1$ à $C_6$; et *m* représente un nombre entier de 0 à 2; ou d'un de ses sels d'addition thérapeutiquement acceptables.

4. Procédé suivant la revendication 1, pour la préparation d'un composé de formule I dans laquelle $R^1$ représente un groupe alkyle inférieur, cyclo-(alkyle inférieur) ou benzyle; $R^2$ représente un groupe oxo ou thioxo, $R^3$ représente un groupe cyano; $R^4$ représente l'hydrogène; $R^9$ représente un groupe alcényle en $C_2$ à $C_6$, 2,3- ou 4-pyridinyle, 2-furannyle, 3-indolyle, 3-thiényle, 4-morpholinyle, phényle facultativement disubstitué avec un groupe alkoxy en $C_1$ à $C_6$; et *m* a la veleur 0 ou 1; ou d'un de ses sels d'addition thérapeutiquement acceptables.

5. Procédé suivant la revendication 1, dans lequel le composé préparé de formule I est
le 1,4-dihydro-2-méthyl-4-oxo-6-[(3-pyridinylméthyl)amino]-5-pyrimidinecarbonitrile;
le 1,4-dihydro-2-méthyl-6-[N-méthyl-N-(3-pyridinylméthyl)amino]-4-oxo-5-pyrimidinecarbonitrile;
le 1,4-dihydro-2-méthyl-4-oxo-6-[(phényléthyl)amino]-5-pyrimidinecarbonitrile;
le 1,4-dihydro-2-méthyl-4-oxo-6-[(2-pyridinyléthyl)amino]-5-pyrimidinecarbonitrile;
le 1,4-dihydro-2-méthyl-4-oxo-6-[(4-pyridinylméthyl)amino]-5-pyrimidinecrbonitrile;
le 1,4-dihydro-2-méthyl-4-oxo-6-[(2-pyridinylméthyl)amino]-5-pyrimidinecarbonitrile;
le 1,4-dihydro-2-méthyl-4-oxo-6-[(phénylméthyl)amino]-5-pyrimidinecarbonitrile;
le 1,4-dihydro-2-méthyl-4-oxo-6-[2-(3-pyridinyl)éthylamino]-5-pyrimidinecarbonitrile;
le 1,4-dihydro-2-méthyl-4-oxo-6-[2-(4-pyridinyl)éthylamino]-5-pyrimidinecarbonitrile;
le 1,4-dihydro-2-méthyl-4-oxo-6-[(3-pyridinyl)amino]-5-pyrimidinecarbonitrile;
le 1,4-dihydro-2-méthyl-6-[(3-pyridinylméthyl)amino]-4-thioxo-5-pyrimidinecarbonitrile;
le 1,4-dihydro-2-éthyl-4-oxo-6-[(3-pyridinylméthyl)amino]-5-pyrimidinecarbonitrile;
le 1,4-dihydro-2-1-méthyléthyl-4-oxo-6-[(3-pyridinylméthyl)amino]-5-pyrimidinecarbonitrile;
le 2-cyclopropyl-1,4-dihydro-4-oxo-6-[(3-pyridinylméthyl)amino]-5-pyrimidinecarbonitrile;
le 1,4-dihydro-2-(1,1-diméthyléthyl)-4-oxo-6-[(3-pyridinylméthyl)amino]-5-pyrimidinecarbonitrile;
le 1,4-dihydro-4-oxo-2-phénylméthyl-6-[(3-pyridinylméthyl)amino]-5-pyrimidinecarbonitrile; ou un de ses sels pharmaceutiquement acceptables.

6. Procédé suivant la revendication 1, dans lequel le composé préparé de formule I est
le 1,4-dihydro-6-[(2-furannylméthyl)amino)]-2-méthyl-4-oxo-5-pyrimidinecarbonitrile;
le 1,4-dihydro-2-méthyl-6-[[2-(4-morpholinyl)éthyl)amino]-4-oxo-5-pyrimidinecarbonitrile;
le 1,4-dihydro-2-méthyl-4-oxo-6-[[2-(3-thiényl)-éthyl]amino]-5-pyrimidinecarbonitrile;
le 1,4-dihydro-6-[[2-(3,4-diméthoxyphényl)éthyl]amino]-2-méthyl-4-oxo-5-pyrimidinecarbonitrile;
le 1,4-dihydro-6-[[2-(1H-indole-3-yl)éthyl]amino]-2-méthyl-4-oxo-5-pyrimidinecarbonitrile;
le 1,4-dihydro-2-méthyl-4-oxo-6-[(2-propényl)amino]-5-pyrimidinecarbonitrile; ou un de ses sels d'addition pharmaceutiquement acceptables.

7. Procédé de préparation d'une compostion pharmaceutique, qui consiste à mettre un composé de formule I suivant la revendication 1, ou un de ses sels thérapeutiquement acceptables, sous une forme convenable pour l'administration thérapeutique.

8. Procédé suivant la revendication 7, dans lequel la composition comprend également un agent couramment utilisé dans le traitement de l'insuffisance cardiaque congestive.

9. Procédé suivant la revendication 8, dans lequel l'agent est choisi entre l'isosorbide dinitrate, le captopril, la nifédipine, l'hydralazine, la prazosine, l'hydrochlorothiazide, le furosémide, la spironolactone, la digitaline et la dobutamine.